# FASCICULE DE BREVET EUROPEEN

(11) **EP 1 668 021 B1**
(45) Date de publication et mention de la délivrance du brevet: **22.06.2011**
(21) Numéro de dépôt: 04787435.9
(22) Date de dépôt: 24.09.2004
(51) Int. Cl.: C07H 17/02, A61K 31/706, A61P 7/02

(54) **NOUVEAUX COMPOSES DU THIOXYLOSE, PROCEDE DE PREPARATION, COMPOSITIONS PHARMACEUTIQUES LES CONTENANT ET LEUR UTILISATION EN THERAPEUTIQUE**
NEUE THIOXYLOSEVERBINDUNGEN, VERFAHREN ZU DEREN HERSTELLUNG, PHARMAZEUTISCHE ZUSAMMENSETZUNGEN, DIE DIESE ENTHALTEN, UND DEREN VERWENDUNG IN THERAPEUTIKA
NOVEL THIOXYLOSE COMPOUNDS, PREPARATION METHOD THEREOF, PHARMACEUTICAL COMPOSITIONS CONTAINING SAME AND USE THEREOF IN THERAPEUTICS

(30) Priorité: 25.09.2003 FR 0311264
(43) Date de publication de la demande: 14.06.2006
(73) Titulaire: LABORATOIRES FOURNIER SA, 21000 Dijon (FR)
(72) Inventeur: BARBEROUSSE, Véronique, F-21121 Hauteville Les Dijon (FR); SAMRETH, Soth, F-21121 Daix (FR); BOUBIA, Benaissa, F-21850 Saint Apollinaire (FR); BELLAMY, François, F-21910 Saulon-La-Rue (FR); PEYROU, Vincent, F-21121 Hauteville Les Dijon (FR)
(74) Mandataire: Gillard, Marie-Louise
(86) Numéro de dépôt international: PCT/FR2004/002409
(87) Numéro de publication internationale: WO 2005/030785

(56) Documents cités:
- US-A- 5 100 913
- US-A- 5 101 048

## Description

La présente invention concerne de nouveaux composés du 5-thioxylose, préférentiellement des dérivés de type 5-thioxylopyranose, ainsi que leur procédé de préparation et leur utilisation en tant que principe actif de médicaments, notamment destinés au traitement ou à la prévention des thromboses.

### Art antérieur

On connaît déjà des dérivés du D-xylose, par exemple dans US 5101048, EP 051 023 B1, US 4 877 808, EP 421 829 B1 ou dans la publication J. Med. Chem. Vol. 36 n° 7, p 898-903. Les composés décrits dans ces documents sont utiles pour réduire les risques de thrombose veineuse chez l'homme. Le mécanisme d'action de ces composés semble être un effet sur les glycosaminoglycanes plasmatiques (J. Biol. Chem., Vol 270 n° 6 p 2662-68, Thromb. Haemost. 1999, 81 p 945-950).

La plupart des composés décrits sont des dérivés résultant du couplage d'un sucre et d'un dérivé aromatique, notamment d'un noyau benzénique diversement substitué. La partie aglycone de ces composés est généralement à caractère hydrophobe. On entend par "partie aglycone" la partie non glucidique de ces composés. En conséquence les composés de l'art antérieur, s'ils présentent l'avantage de posséder une bonne activité lorsqu'ils sont administrés par voie orale, ne sont pratiquement pas utilisables lorsqu'une administration par voie injectable est préférable ou ne peut pas être évitée.

### Objet de l'invention

On a maintenant découvert que des dérivés du thioxylose, dont l'aglycone présente un caractère plus hydrophile, ont une bonne activité antithrombotique, et sont susceptibles d'être administrés soit par voie orale, soit par voie injectable.

### Description

Les nouveaux composés selon l'invention sont caractérisés en ce qu'ils sont choisis parmi :
a) les composés de formule : 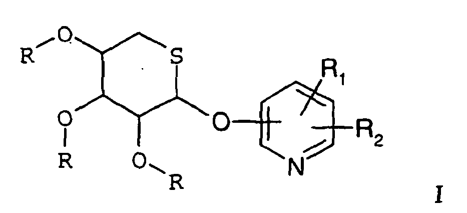 dans laquelle :
   - le groupe pentapyranosyle représente un groupe 5-thio-β-D-xylopyranosyle ou un groupe 5-thio-β-L-xylopyranosyle,
   - R représente un atome d'hydrogène, un groupe acyle en C₂-C₆, un groupe acétyle substitué par un hétérocycle azoté ou un groupe -COOR',
   - R₁ et R₂ représentent chacun, indépendamment l'un de l'autre, un atome d'hydrogène, un atome d'halogène ou un groupe cyano, nitro, trifluorométhyle, un groupe alkyle en C₁-C₄ éventuellement substitué par un noyau aromatique, un groupe COOR', un groupe -CH₂-NR'R", un groupe alcoxy en C₁-C₄, un groupe -NH-CO-R' , ou un groupe -NH-SO₂-R',
   - R' et R" représentent chacun indépendamment un groupe alkyle en C₁-C₄,
b) leurs sels d'addition, oxydes ou sels d'ammonium quaternaires.

L'invention a également pour objet un procédé pour la préparation des composés de formule I ainsi que de leurs sels d'addition, oxydes ou sels d'ammonium quaternaire.

L'invention concerne également les composés de formule I pour leur utilisation en tant que substance pharmacologiquement active.

En particulier, l'invention concerne l'utilisation d'au moins une substance choisie parmi les composés de formule I et leurs sels d'addition, oxydes ou sels d'ammonium quaternaire non toxiques pour la préparation d'un médicament, utile en thérapeutique humaine ou animale, destiné à la prévention ou au traitement des thromboses, notamment les thromboses veineuses. Les composés selon l'invention étant actifs selon un mode d'action faisant intervenir les glycosaminoglycanes, ils pourront être utiles en tant que principe actif d'un médicament destiné au traitement ou à la prévention de toute autre maladie dans laquelle les glycosaminoglycanes sont impliqués.

### Description détaillée

Dans la formule I, on entend par groupe alkyle en C₁-C₄ une chaîne hydrocarbonée linéaire, ramifiée ou cyclisée ayant de 1 à 4 atomes de carbone. Des exemples de groupes alkyle en C₁-C₄ sont notamment les groupes méthyle, éthyle, propyle, butyle, 1-méthyléthyle, 1,1-diméthyléthyle, 1-méthylpropyle, 2-méthylpropyle, cyclopropyle ou cyclopropylméthyle.

Par groupe alkyle éventuellement substitué par un noyau aromatique, on entend par exemple un groupe phénylméthyle (benzyle) ou phényléthyle.

Par halogène, il faut comprendre un atome de fluor, de chlore, de brome ou d'iode, et préférentiellement un atome de chlore, de fluor ou de brome.

Par groupe acyle en C₂-C₆, on désigne les groupes acétyle, propanoyle, butanoyle, pentanoyle, hexanoyle, ainsi que leurs homologues dans lesquels la chaîne peut être ramifiée.

On entend par groupe alcoxy en C₁-C₄ une chaîne hydrocarbonée linéaire, ramifiée ou cyclisée ayant de 1 à 4 atomes de carbone liée par un atome d'oxygène. A titre d'exemples de groupes alcoxy en C₁-C₄ on peut citer les groupes méthoxy, éthoxy, propoxy, butoxy, 1-méthyléthoxy, 1,1-diméthyléthoxy, 1-méthylpropoxy, 2-méthylpropoxy ou cyclopropylméthoxy.

Par groupe acétyle substitué par un hétérocycle azoté, on entend par exemple un groupe (1-pipéridinyl)acétyle ou un groupe (1-pyrolidinyl)acétyle.

Par oxydes, on entend les dérivés N-oxydes du noyau pyridine.

Par sels d'ammonium quaternaire, on entend les sels d'ammonium issus de la réaction de l'azote de la pyridine avec par exemple un composé halogéné, ladite réaction conduisant à la formation d'un halogénure de pyridinium.

Par sels d'addition, on entend les sels d'addition obtenus par réaction d'un composé de formule I avec un acide minéral ou organique. De préférence, il s'agit des sels d'addition pharmaceutiquement acceptables. Les hydrates ou solvates des composés de formule I ou des sels des composés de formule I font également partie intégrante de l'invention.

Parmi les acides minéraux convenant pour salifier un composé basique de formule I, on préfère les acides chlorhydrique, bromhydrique, phosphorique et sulfurique. Parmi les acides organiques convenant pour salifier un composé basique de formule I, on préfère les acides méthanesulfonique, benzènesulfonique, toluènesulfonique, maléïque, fumarique, oxalique, citrique, tartrique, lactique et trifluoroacétique.

Parmi les composés de formule I ci-dessus, on préfère les composés dans lesquels :
- le groupe pentapyranosyle représente un groupe 5-thio-β-D-xylopyranosyle ou un groupe 5-thio-β-L-xylopyranosyle,
- R représente un atome d'hydrogène, un groupe acyle en C₂-C₆, ou un groupe -COOR',
- R' représente un groupe alkyle en C₁-C₃,
- R₁ et R₂ représentent chacun, indépendamment l'un de l'autre, un atome d'hydrogène, un atome d'halogène ou un groupe cyano, nitro, trifluorométhyle, ou un groupe alkyle en C₁-C₄ éventuellement substitué par un noyau aromatique.

Parmi les composés selon la présente invention, on préfère tout particulièrement ceux dans lesquels R₁ et R₂ représentent un atome d'hydrogène et le groupe pentapyranosyle est le 5-thio-β-D-xylopyranosyle.

Parmi les composés selon la présente invention, on préfère également les composés dans lesquels R est l'atome d'hydrogène, le groupe -COCH₃, le groupe -COOCH₃ ou le groupe -COOC₂H₅.

Parmi les composés selon la présente invention, on préfère tout particulièrement ceux dans lesquels le groupe pentapyranosyle est en position 3 de l'hétérocycle pyridine.

Les composés de formule 1 selon l'invention peuvent être préparés en mettant en oeuvre les méthodes de glycosilation connues de l'homme de métier, notamment :
a) la méthode de HELFERICH décrite dans l'ouvrage «The Carbohydrate, Chemistry and Biochemistry », 2ème édition, Academic Press, New-York-Londres 1972, Tome IA pages 292-294, par condensation d'un sucre peracétylé avec un hydroxyhétérocycle aromatique en présence d'un acide de Lewis ;
b) la méthode de KOENIGS-KNORR (idem, pages 295-299) par condensation d'un acylose halogéné avec un groupe hydroxy à caractère phénolique en présence d'un accepteur de protons, tels que le cyanure mercurique, l'imidazolate d'argent ou le trifluorométhylsulfonate d'argent.
c) La méthode de SCHMIDT par condensation d'un trichloracétimidate d'osyle avec un hydroxyhétérocycle aromatique, en présence d'un acide de Lewis, tel que par exemple le trifluorométhanesulfonate de triméthylsilyle ou l'éthérate de trifluorure de bore.

Les composés de formule 1 sont préparés de préférence selon des méthodes dérivées des procédés référencés ci-dessus.

Selon un premier procédé général, on effectue les étapes consistant à :
a) faire réagir un pyridinol de formule : 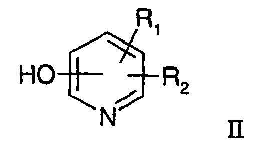 dans laquelle :
   R₁ et R₂ représentent chacun, indépendamment l'un de l'autre, un atome d'hydrogène, un atome d'halogène ou un groupe cyano, nitro, trifluorométhyle, un groupe alkyle en C₁-C₄ éventuellement substitué par un noyau aromatique, un groupe COOR', un groupe -CH₂-NR'R", un groupe alcoxy en C₁-C₄, un groupe -NH-CO-R', ou un groupe -NH-SO2-R',
   R'et R" représentent chacun indépendamment un groupe alkyle en C₁-C₄, avec un dérivé du 5-thioxylopyranose de formule : 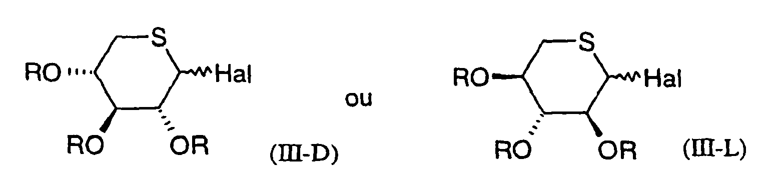
   dans laquelle Hal représente un halogène, préférentiellement le brome et R représente un groupe acyle en C₂-C₆, préférentiellement le groupe acétyle, dans un solvant aprotique tel que l'acétonitrile ou le toluène, en présence d'un sel d'argent, notamment l'oxyde ou l'imidazolate d'argent ou d'un sel de zinc (notamment l'oxyde ou le chlorure) en milieu anhydre, à une température comprise entre 25 et 80 °C et pendant 1 à 10 heures, pour obtenir le composé de formule : 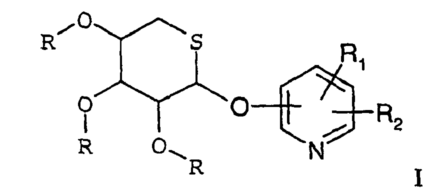 dans laquelle le groupe pentapyranose représente le D- ou le L-5-thioxylopyranose, et R, R₁, R₂ conservent la même signification que dans les composés de départ ;
b) si nécessaire, faire réagir le composé de formule I obtenu ci-dessus avec une solution d'ammoniac dans du méthanol pour réaliser la désacylation et ainsi remplacer le groupe acyle par des atomes d'hydrogène et obtenir le composé de formule : 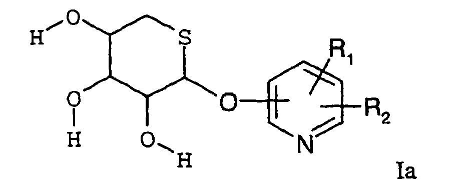 dans laquelle R₁ et R₂ conservent la même signification que ci-dessus ;
c) si nécessaire, faire réagir l'un des composés I ou Ia obtenus ci-dessus avec un acide selon des méthodes connues de l'homme de métier pour obtenir le sel d'addition correspondant.

En variante de l'étape b) décrite ci-dessus, le remplacement du groupe acyle par un atome d'hydrogène peut être réalisé par action d'un alcoolate métallique, préférentiellement le méthylate de sodium, dans le méthanol, à une température comprise entre 0 et 30 °C et pendant 0,5 à 2 heures pour obtenir le composé de formule Ia à partir du composé de formule I dans laquelle R représente un groupe acyle en C₂-C₆.

Selon un second procédé, les composés de formule I peuvent être obtenus par action du tétra-O-acétyl-5-thioxylopyranose de formule : 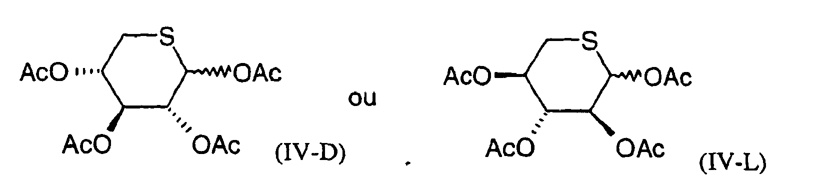 dans laquelle Ac représente le groupe acétyle avec un composé de formule : 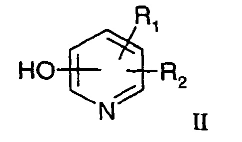 dans laquelle :
R₁ et R₂ représentent chacun, indépendamment l'un de l'autre, un atome d'hydrogène, un atome d'halogène ou un groupe cyano, nitro, trifluorométhyle, un groupe alkyle en C₁-C₄ éventuellement substitué par un noyau aromatique, un groupe COOR', un groupe -CH₂-NR'R", un groupe alcoxy en C₁-C₄, un groupe -NH-CO-R', ou un groupe -NH-SO₂-R',
R'et R" représentent chacun indépendamment un groupe alkyle en C₁-C₄, dans un solvant aprotique, tel que par exemple le dichlorométhane, en présence d'un catalyseur de type acide de Lewis, par exemple le tétrachlorure d'étain, à une température comprise entre 20 et 60 °C et pendant 1 à 2 heures, pour obtenir le composé de formule : 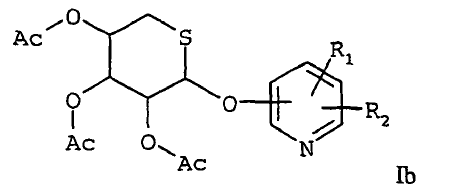 dans laquelle R₁ et R₂ conservent la même signification que dans les composés de départ.
Le composé de formule Ib peut ensuite être mis en réaction selon le protocole décrit dans le procédé précédent pour obtenir le composé pyranosyle non substitué et/ou le sel avec un acide.

Selon un autre mode de préparation des composés de formule I, on fait réagir un dérivé du thioxylose de formule : 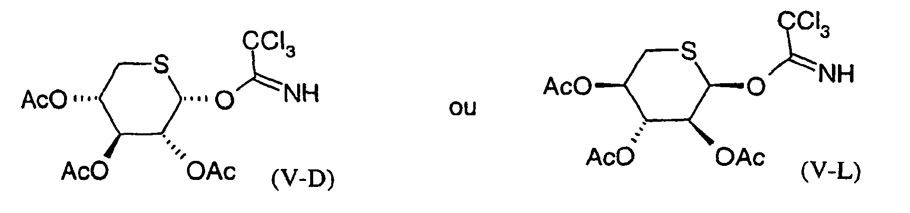 dans laquelle Ac représente le groupe acétyle,
avec un composé de formule : 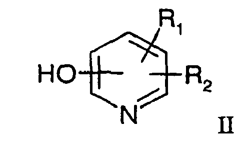 dans laquelle :
R₁ et R₂ représentent chacun, indépendamment l'un de l'autre, un atome d'hydrogène, un atome d'halogène ou un groupe cyano, nitro, trifluorométhyle, un groupe alkyle en C₁-C₄ éventuellement substitué par un noyau aromatique, un groupe COOR', un groupe -CH₂-NR'R", un groupe alcoxy en C₁-C₄, un groupe -NH-CO-R', ou un groupe -NH-SO₂-R',
R'et R" représentent chacun indépendamment un groupe alkyle en C₁-C₄,
dans un solvant aprotique, tel que le dichlorométhane, en présence d'un catalyseur tel que le trifluorométhanesulfonate de triméthylsilyle, à une température comprise entre -25°C et la température ambiante et pendant 1 à 5 heures pour obtenir le thioxylopyranoside de formule : 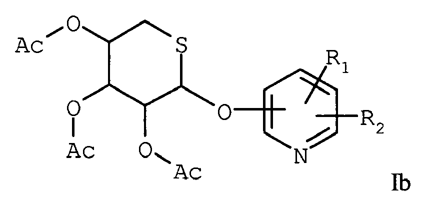 dans laquelle R₁ et R₂ conservent la même signification que dans les composés de départ.

Le composé de formule Ib ainsi obtenu peut être ensuite mis en réaction comme précédemment pour obtenir les composés pyranosyle non substitué et/ou les sels d'acide.

Les composés de formule I dans laquelle R représente un groupe COOR' peuvent être obtenus à partir des composés de formule Ia par action d'un pyrocarbonate de formule : 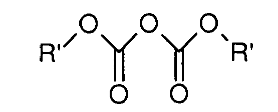 dans laquelle R' représente un groupe alkyle en C₁-C₄, la réaction étant conduite dans un solvant aprotique, tel que par exemple l'acétonitrile et en présence d'une base aprotique, telle que par exemple 1a 4-(diméthylamino)pyridine (DMAP).

Les dérivés N-oxydes peuvent être obtenus par couplage d'un N-oxyde pyridinol de formule IIa : 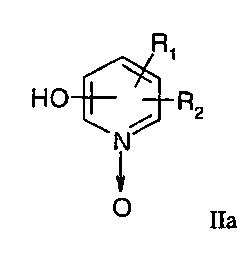 avec un 5-thioxylopyranose de formule III-D ou III-L selon le mode opératoire décrit précédemment pour le premier procédé général.

Les sels d'ammonium quaternaire peuvent être obtenus par réaction d'un composé de formule I avec un halogénure organique convenable selon les modes opératoires classiques bien connus de l'homme du métier.

D'une façon générale on préfère utiliser le bromure de 2,3,4-tri-O-acétyl-5-thio-α-D-xylopyranosyle ou le tétra-O-acétyl-5-thio-α-D-xylopyranose quand il s'agit d'obtenir un dérivé du β-D-5-thio-xylopyranose

Les réactions de glycosylation décrites précédemment conduisent le plus souvent à un mélange des isomères de configuration α et β et il est généralement nécessaire d'optimiser les conditions opératoires afin d'obtenir des proportions favorables à l'isomère de configuration β. Pour cette raison, il peut être nécessaire d'effectuer des purifications soit par recristallisation, soit par chromatographie, pour obtenir l'isomère β pur.

Les exemples suivants ont pour but d'illustrer l'invention, et ne sauraient en aucun cas en limiter la portée. Les points de fusion sont mesurés au banc Kofler et les valeurs spectrales de Résonance Magnétique Nucléaire sont caractérisées par le déplacement chimique calculé par rapport au TMS, par le nombre de protons associés au signal et par la forme du signal (s pour singulet, d pour doublet, t pour triplet, q pour quadruplet, m pour multiplet). La fréquence de travail et le solvant utilisé sont indiqués pour chaque composé.

### Préparation 1

### N-(5-méthoxy-2-pyridinyl)méthanesulfonamide

On refroidit à 0°C une solution de 10,7 g (86 mM) de 5-méthoxy-2-pyridinamine dans 55 ml de dichlorométhane et on ajoute doucement 72 ml (144 mM) d'une solution 2M d'anhydride de l'acide méthanesulfonique dans le dichlorométhane. Le mélange réactionnel est agité pendant 6 heures à température ambiante, puis on ajoute une solution de bicarbonate de sodium en quantité suffisante pour obtenir pH 9 environ. Le mélange obtenu est concentré sous pression réduite et le résidu est repris dans 160 ml d'éthanol à 50°C, le mélange obtenu est séché, filtré et le filtrat est concentré sous pression réduite. Le résidu est trituré dans 50 ml d'éthanol froid et le solide obtenu est filtré et rincé avec un peu d'éthanol sur le filtre, puis séché. On obtient ainsi 8,7 g du produit attendu sous forme d'une poudre blanche (rendement = 50%).
F = 156-158 °C.

### Préparation II

### N-(5-hydroxy-2-pyridinyl)méthanesulfonamide

On prépare une solution de 8,7 g (43 mM) du composé obtenu selon la préparation I dans 90 ml de dichlorométhane et on ajoute doucement 12,2 ml (129 mM) de tribromure de bore. Le mélange réactionnel est agité pendant 3 heures à reflux du solvant, puis concentré sous pression réduite ; on ajoute 200 ml d'eau au résidu d'évaporation et on neutralise le mélange obtenu par addition d'une solution d'hydroxyde de sodium en quantité suffisante pour obtenir pH 7environ. Le précipité obtenu est séparé par filtration et rincé à l'eau sur le filtre, puis trituré dans 250 ml d'éthanol chaud ; après refroidissement, le solide est filtré et rincé par 60 ml d'éthanol sur le filtre, puis séché. On obtient ainsi 6,55 g du produit attendu sous forme d'une poudre blanche (rendement = 80%).
F = 246-247 °C.

### Préparation III

### 4-(trifluorométhyl)-3-pyridinol

On refroidit à -5°C une solution de 2 g (12,34 mM) de 3-amino-4-(trifluorométhyl)pyridine dans 28 ml d'acide sulfurique à 50 %, et on ajoute lentement une solution de 1,03 g (14,8 mM) de nitrite de sodium dans 10 ml d'eau. On laisse la température du mélange revenir à la température ambiante et on continue d'agiter le mélange pendant 30 mn. On ajoute ensuite 25 ml d'acide sulfurique concentré et on agite le mélange réactionnel à 100-110 °C pendant 2 heures. Après refroidissement, on neutralise le milieu réactionnel par ajout d'une solution de bicarbonate de sodium saturée jusqu'à pH 6-7 et on extrait par l'acétate d'éthyle. La phase organique obtenue est lavée à l'eau, séchée sur sulfate de magnésium et concentrée sous pression réduite. On obtient ainsi 1,92 g du composé attendu sous forme d'une poudre marron (rendement = 95 %)
F = 112-114 °C.

### Préparation IV

### 6-cyano-3-pyridinol

On refroidit à -0°C une solution de 1 g (8,06 mM) de 3-amino-6-cyanopyridine dans 12 ml d'eau et 1,2 ml d'acide sulfurique, et on ajoute lentement 1 g (14,5 mM) de nitrite de sodium. On laisse la température du mélange revenir à la température ambiante puis on agite le mélange réactionnel à 100 °C pendant 3 heures. Le mélange réactionnel est abandonné pendant une nuit au réfrigérateur. Le précipité formé est séparé par filtration et lavé à l'eau glacée. Le produit attendu également présent dans le filtrat est extrait par l'acétate d'éthyle et joint au précipité précédent. Ce produit brut est purifié par chromatographie sur gel de silice en éluant à l'aide d'un mélange dichlorométhane/méthanol (98/2 ;v/v). On obtient ainsi 613 mg du composé attendu sous forme d'une poudre marron (rendement = 63 %)
F = 191-194 °C.

### Préparation V

### 6-cyano-5-fluoro-3-pyridinol

On prépare un mélange de 2,94 g (20 mM) de 6-chloro-5-fluoro-3-pyridinol, 360 mg (0,4 mM) de tris(benzylidèneacétone)dipalladium(0), 440 mg (0,8 mM) de 1,1'-bis(diphénylphosphino)ferrocène, 156 mg (2,4 mM) de poudre de zinc, 1,4 g (12 mM) de cyanure de zinc dans 40 ml de N,N-diméthylacétamide, puis on agite le mélange réactionnel à 140 °C pendant 5 heures. Le mélange réactionnel est abandonné pendant une nuit à température ambiante. On ajoute lentement 40 ml d'ammoniaque 2N puis on ajuste le pH à environ 4,5 avec de l'acide chlorhydrique 5N. Le mélange est extrait 3 fois par 100 ml d'acétate d'éthyle, la phase organique obtenue est séchée sur sulfate de magnésium et concentrée sous pression réduite. Le résidu d'évaporation est purifié par chromatographie sur gel de silice en éluant à l'aide d'un mélange méthylcyclohexane/acétate d'éthyle (en gradient de 8/2 à 5/5 ;v/v). On obtient ainsi 1,8 g du composé attendu sous forme d'une huile orange (rendement = 67 %)
RMN (300 MHz, DMSO) δ= 8.16 (s, 1 H,), 7.32 (d, 1H,)

### Préparation VI

### 6-cyano-2-méthyl-3-aminopyridine

On prépare un mélange de 3,5 g (18,6 mM) de 6-bromo-2-méthyl-3-aminopyridine, 352 mg (0,37 mM) de tris(benzylidèneacétone)dipalladium(0), 532 mg (0,92 mM) de 1,1'-bis(diphénylphosphino)ferrocène, 14-6 mg (2,2 mM) de poudre de zinc, 1,4 g (12 mM) de cyanure de zinc dans 70 ml de N,N-diméthylacétamide, puis on agite le mélange réactionnel à 20 °C pendant 22 heures. Le mélange réactionnel est dilué par 200 ml d'acétate d'éthyle et lavé par une solution de chlorure d'ammonium 2N. (la décantation n'est obtenue qu'après filtration du mélange sur adjuvant de filtration de type célite). La phase organique obtenue est lavée par une solution de chlorure de sodium, séchée sur sulfate de magnésium et concentrée sous pression réduite. Le résidu d'évaporation est purifié par chromatographie sur gel de silice en éluant à l'aide d'un mélange dichlorométhane/acétate d'éthyle (9/1 ;v/v). On obtient ainsi 1,6 g du composé attendu sous forme d'une poudre jaune (rendement = 65 %)
F = 192-196 °C.

### Préparation VII

### 6-cyano-2-méthyl-3-pyridinol

En opérant de façon analogue à la préparation IV, au départ du composé obtenu selon la préparation VI, on obtient le composé attendu sous forme d'une poudre jaune (rendement = 50 %).
F = 201-205 °C

### Préparation VIII

### 5-fluoro-3-pyridinol, N-oxyde

On chauffe à 70 °C une solution de 1,5 g (13,26 mM) de 5-fluoro-3-pyridinol dans 8 ml d'acide acétique et on ajoute doucement 1,25 ml (14,6 mM) d'eau oxygénée à 35 %. Le mélange réactionnel est agité pendant 15 heures à 70 °C, puis concentré sous pression réduite ; on ajoute 50 ml d'eau froide au résidu d'évaporation et on agite pendant 1 heure. Le précipité obtenu est séparé par filtration et rincé à l'eau et avec un minimum d'acétate d'éthyle froid sur le filtre, puis séché. On obtient ainsi 930 mg du produit attendu sous forme d'une poudre blanche (rendement = 55%).
F = 197 °C.

### Préparation IX

### S-fluoro-2-cyano-3-pyridinol

On chauffe à doux reflux pendant 16 heures une solution de 890 mg (6,89 mM) du composé obtenu selon la préparation VIII, 1,74 g (17,23 mM) de triéthylamine, 2,39 g (24,1 mM) de cyanure de triméthylsilyle dans 5 ml d'acétonitrile. Le mélange réactionnel est concentré sous pression réduite et on ajoute 50 ml d'acétate d'éthyle au résidu d'évaporation. La phase organique obtenue est lavée avec une solution de chlorure de sodium puis séchée sur sulfate de magnésium et concentrée sous pression réduite. Le résidu d'évaporation est purifié par chromatographie sur gel de silice en éluant à l'aide d'un mélange dichlorométhane/méthanol (98/2 ;v/v). Le produit huileux issu de la chromatographie est trituré dans l'éther éthylique et laisse cristalliser le composé attendu sous forme d'un solide blanc écru.(rendement = 18 %)
F = 201°C.

### Exemple 1

### 3-pyridinyl 2,3,4-tri-O-acétyl-5-thio-β-D-xylopyranoside

On mélange 2,15 g (15,8 mM) de chlorure de zinc anhydre, 1g (10,5 mM) de 3-hydroxypyridine et 3 g de tamis moléculaire 13X dans 10 ml de toluène et 10 ml d'acétonitrile. Le mélange est agité pendant 15 mn puis chauffé à 60 °C et placé à l'abri de la lumière. On ajoute 2,2 g (12,6 mM) d'imidazolate d'argent et 4,5 g (12,6 mM) de bromure de 2,3,4-tri-O-acétyl-5-thio-α-D-xylopyranosyle. Le mélange est agité pendant 18 heures à 60 °C puis filtré. On ajoute 50 ml d'acétate d'éthyle au filtrat et la phase organique est lavée avec une solution de soude 1N (on observe une floculation qui est éliminée par filtration sur un lit d'adjuvant de filtration). La phase organique séparée est lavée à l'eau puis séchée sur sulfate de magnésium et concentrée sous pression réduite. Le résidu est cristallisé par addition d'éther éthylique ; les cristaux obtenus sont filtrés et séchés. On obtient ainsi 1,45 g du produit attendu sous forme de cristaux blancs (rendement = 37 %). F = 147°C
[α]²⁴ D = - 84 ° (c = 0,10 ; CHCl₃)

### Exemple 1A

### 3-pyridinyl 2,3,4-tri-O-acétyl-5-thio-β-D-xylopyranoside, méthanesulfonate

On dissout 0,5 g (1,35 mM) du composé obtenu selon l'exemple 1 dans 100 ml d'acétate d'éthyle et on ajoute 50 ml d'éther éthylique dans et une solution de 0,13 g (1,35 mM) d'acide méthanesulfonique dans 1 ml de dichlorométhane et 0,2 ml de méthanol. Le mélange réactionnel est agité pendant 10 minutes et le précipité est séparé de la phase liquide puis trituré dans l'éther éthylique. Les cristaux formés sont filtrés, rincés et séchés. On obtient ainsi 0,5 g du sel attendu, sous forme de cristaux blancs (rendement = 80 %).
F = 104 °C
[α]²⁰_{D}= - 82 ° (c = 0,32 ; CH₃OH)

### Exemple 2

### 3-pyridinyl 5-thio-β-D-xylopyranoside

On mélange 1,35 g (3,6 mM) du composé obtenu selon l'exemple 1 avec 50 ml d'une solution 7 moles/l d'ammoniac dans le méthanol et on agite ce milieu réactionnel pendant 2 heures à température ambiante. Le mélange est ensuite concentré sous pression réduite et les cristaux obtenus sont repris dans 30 ml de méthanol à reflux. Après refroidissement à 10 °C, les cristaux sont séparés par filtration et séchés. On obtient ainsi 0,75 g du produit attendu sous forme de cristaux blancs (rendement = 84 %).
F = 219 °C
[α]²²_{D}= - 99 ° (c = 0,445 ; DMSO)

### Exemple 2A

### 3-pyridinyl 5-thio-β-D-xylopyranoside, méthanesulfonate

On prépare une suspension de 10 g (41,1 mM) du composé obtenu selon l'exemple 2 dans 100 ml de méthanol et on ajoute rapidement une solution de 2,8 ml (4,15 g ; 43,1 mM) d'acide méthanesulfonique dans 40 ml de méthanol. Le mélange est agité pendant 2 heures à température ambiante. II se forme une solution puis un précipité. Le solide obtenu est séparé par filtration et recristallisé dans environ 200 ml d'un mélange méthanol-eau (95/5). On obtient ainsi 9 g du sel attendu, sous forme d'une poudre beige (rendement = 64 %).
F = 168°C
[α]²⁹_{D}= - 110 ° (c = 0,3 ; H₂O)

### Exemple 2B

### 3-pyridinyl 5-thio-β-D-xylopyranoside, sulfate neutre

On prépare une suspension de 0,5 g (2,05 mM) du composé obtenu selon l'exemple 2 dans 2 ml de méthanol et on ajoute 30 ml d'eau et 52,5 µl d'acide sulfurique. Le mélange est agité pendant 15 mn à température ambiante puis congelé et lyophilisé. Le lyophilisat obtenu est repris dans 25 ml d'eau et à nouveau lyophilisé. On obtient ainsi le produit attendu sous forme d'une poudre blanche (rendement = 95%)
F = 80°C
[α]²⁸_{D} = - 48,8° (c = 0,5 ; DMSO)

### Exemple 2C

### 3-pyridinyl 5-thio-β-D-xylopyranoside, chlorhydrate

On prépare une suspension de 5 g (20,55 mM) du composé obtenu selon l'exemple 2 dans 20,5 ml d'une solution N de chlorure d'hydrogène dans l'éther éthylique. On ajoute ensuite 2 ml de méthanol. Il se forme une gomme qui cristallise. Le mélange et agité pendant 1 heure, puis le précipité est séparé par filtration, séché et recristallisé dans de l'éthanol à 95 %. Après séchage on obtient le composé attendu (contient une mole d'éthanol pour une mole de sel) sous forme de cristaux beiges (rendement = 90 %). Le produit est repris en solution dans l'eau et lyophilisé. On obtient ainsi le produit attendu sous forme d'une poudre beige.
F = 120 °C
[α]²³_{D} = - 149 ° (c = 0,2 ; H₂O)

### Exemple 2D

### 3-pyridinyl 5-thio-β-D-xylopyranoside, bromhydrate

En opérant de façon analogue à l'exemple 2C, en ajoutant le bromure d'hydrogène en solution dans l'éther éthylique à une suspension du composé selon l'exemple 2 dans le méthanol, on obtient le produit attendu sous forme d'une poudre beige.
(Rendement = 30%)
[α]²⁸_{D}= - 73 ° (c = 0,57; DMSO)

### Exemple 3

### 3-pyridinyl 2,3,4-tri-O-acétyl-5-thio-β-L-xylopyranoside

En opérant de façon analogue à l'exemple 1, au départ du bromure de 2,3,4-tri-O-acétyl-5-thio-a-L-xylopyranosyle, et après purification par chromatographie sur gel de silice en éluant à l'aide d'acétate d'éthyle, on obtient le produit attendu sous forme d'une huile (rendement = 10 %).
RMN ¹H (300 MHz ; CDCl₃) δ = 8,40 (d, 1H) ; 8,33 (dd, 1H) ; 7,38 (m, 1H) ; 7,26 (m, 1H) ; 5,52 (t, 1H) ; 5,14 (m, 3H) ; 3,00 (m, 1H) ; 2,68 (m, 1H) ; 2,06 (m, 9H).

### Exemple 4

### 3-pyridinyl 5-thio-β-L-xylopyranoside

En opérant de façon analogue à l'exemple 2, au départ du composé obtenu selon l'exemple 3, on obtient le composé attendu sous forme d'une poudre blanc cassé (rendement = 27 %).
F = 216 °C
[α]³²_{D}= + 95 ° (c = 0,36 ; DMSO)

### Exemple 5

### 2-pyridinyl 2,3,4-tri-O-acétyl-5-thio-β-D-xylopyranoside

On prépare une solution de 0,5 g (5,2 mM) de 2-hydroxypyridine dans 20 ml de dichlorométhane et on ajoute 2,5 g (5,7 mM) de trichloroacétimidate de 2,3,4-tri-O-acétyl-5-thio-α-D-xylopyranosyle. Le mélange est refroidi à - 20 °C et on ajoute, sous agitation, 2,7 ml d'une solution 0,2 M de trifluorométhanesulfonate de triméthylsilyle. Le milieu réactionnel est maintenu sous agitation pendant 4 heures à 0 °C, puis on ajoute 0,5 ml de diisopropyléthylamine, et on concentre sous pression réduite. Le résidu d'évaporation est cristallisé par addition d'un mélange éther éthylique/méthylcyclohexane. Le produit brut est isolé et recristallisé dans un mélange méthanol-eau. On obtient ainsi 1,07 g du produit attendu sous forme d'une poudre beige (rendement = 55 %).
F=138°C
[α]³²_{D} = - 66 ° (c = 0,38 ; CHOCl₃)

### Exemple 6

### 2-pyridinyl 5-thio-β-D-xylopyranoside

En opérant de façon analogue à l'exemple 2, au départ du composé obtenu selon l'exemple 5, on obtient le produit attendu sous forme de cristaux blancs (rendement = 78 %).
F=196°C
[α]²³_{D} = - 73 ° (c = 0,46 ; DMSO)

### Exemple 7

### pyridin-3-yl N-oxyde 2,3,4-tri-O-acétyl-5-thio-β-D-xylopyranoside

En opérant de façon analogue à l'exemple 1, au départ du N-oxyde du 3-pyridinol, on obtient le produit attendu sous forme d'une poudre beige clair (rendement = 10 %).
F = 161 °C
[α]²⁸_{D} = - 96 ° (c = 0,58 ; CHCl₃)

### Exemple 8

### pyridin-3-yl N-oxyde 5-thio-β-D-xylopyranoside

On dissout 2,65 g (6,9 mM) du compose obtenu selon l'exemple 7 dans 50 ml de méthanol et on ajoute, à température ambiante, 0,265 ml d'une solution de méthylate de sodium à 8 % dans le méthanol. Le mélange réactionnel est agité pendant 30 mn. On ajoute de l'eau de façon à dissoudre le précipité formé, puis on ajoute de la résine IR 120 (forme acide) de façon à amener le pH du mélange à environ 6. La résine est ensuite éliminée par filtration et le filtrat est concentré sous pression réduite. Le résidu obtenu est cristallisé dans le méthanol, séparé par filtration puis dissout dans 40 ml d'eau à chaud. La solution est filtrée puis lyophilisée. On obtient ainsi 1,48 g du composé attendu sous forme d'un solide fin blanc (rendement = 83 %).
F=98°C
[α]²⁸_{D}= - 90 ° (c = 0,48 ; DMSO)

### Exemple 9

### 4-pyridinyl 2,3,4-tri-O-acétyl-5-thio-β-D-xylopyranoside

En opérant de façon analogue à l'exemple 1, au départ de 4-hydroxypyridine, on obtient le produit attendu sous forme de cristaux beiges (rendement = 12 %).
F = 152-158 °C
[α]²⁰_{D}= - 73 ° (c = 1,00 ; CHCl₃)

### Exemple 10

### 4-pyridinyl 5-thio-β-D-xylopyranoside

En opérant de façon analogue à l'exemple 8, au départ du composé obtenu selon l'exemple 9, on obtient le produit attendu sous forme d'une poudre blanc cassé (rendement = 59 %).
F = 165-168 °C
[α]²⁰_{D} = - 94 ° (c = 1,00 ; DMSO)

### Exemple 11

### 2-méthyl-3-pyridinyl 2,3,4-tri-O-acétyl-5-thio-β-D-xylopyranoside

On mélange 6 g (73,7 mM) d'oxyde de zinc, 2,6 g (19 mM) de chlorure de zinc et 4 g de tamis moléculaire 4Å. On dessèche parfaitement ce mélange puis on ajoute 2 g (18,3 mM) de 2-méthyl-3-pyridinol et 6,6 g (18,6 mM) de bromure de 2,3,4-tri-O-acétyl-5-thio-α-D-xylopyranosyle dans 40 ml de toluène et 40 ml d'acétonitrile. Le mélange est agité à 60 °C pendant 18 heures puis filtré. Le filtrat est concentré sous pression réduite et le résidu d'évaporation est purifié par chromatographie sur gel de silice en éluant à l'aide d'un mélange acétate d'éthyle/hexane (7/3 puis 100 % acétate d'éthyle ; v/v). On obtient ainsi le produit attendu sous forme d'un solide beige (rendement = 7 %).
RMN ¹H (300 MHz ; CDCl₃) 2,04 (s, 3H) ; 2,06 (s, 3H) ; 2,07 (s, 3H) ; 2,42 (s, 3H) ; 2,70 (dd, 1H) ; 2,98 (dd, 1H) ; 5,16 (m, 3H) ; 5,77 (t, 1H) ; 7,12 (m, 1H) ; ; 7,37 (d, 1H) ; 8,19 (m, 1H).

### Exemple 12

### 2-méthyl-3-pyridinyl 5-thio-β-D-xylopyranoside

En opérant de façon analogue à l'exemple 2, au départ du composé obtenu selon l'exemple 11, on obtient le produit attendu sous forme d'un solide fin beige (rendement = 98 %).
F=187°C
[α]²³_{D}= - 84 ° (c = 0,2 ; DMSO)

### Exemple 13

### 2-(phénylméthyl)-3-pyridinyl 2,3,4-tri-O-acétyl-5-thio-β-D-xylopyranoside

En opérant de façon analogue à l'exemple 1, au départ de 2-(phénylméthyl)-3-pyridinol, on obtient le produit attendu sous forme d'un solide blanc (rendement = 25 %).
F = 163-166 °C
[α]²⁰_{D}= - 104 ° (c = 1,00 ; CHCl₃)

### Exemple 14

### 2-(phénylméthyl)-3-pyridinyl 5-thio-β-D-xylopyranoside

En opérant de façon analogue à l'exemple 2, au départ du composé obtenu selon l'exemple 13, on obtient le produit attendu sous forme d'une poudre blanc cassé (rendement = 54 %).
F = 156 °C
[α]²⁰_{D}= - 97 ° (c = 1,00 ; DMSO)

### Exemple 15

### 2-éthyl-6-méthyl-3-pyridinyl 2,3,4-tri-O-acétyl-5-thio-β-D-xylopyranoside

En opérant de façon analogue à l'exemple 1, au départ de 2-éthyl-6-méthyl-3-pyridinol, on obtient le produit attendu sous forme d'une poudre blanche (rendement = 19 %).
F = 123-127 °C
[α]²⁰_{D}= - 84 ° (c = 1,00 ; CHCl₃)

### Exemple 16

### 2-éthyl-6-méthyl-3-pyridinyl 5-thio-β-D-xylopyranoside

En opérant de façon analogue à l'exemple 8, au départ du composé obtenu selon l'exemple 15, on obtient le produit attendu sous forme d'une poudre blanche (rendement = 58 %).
F = 172-174 °C
[α]²⁰_{D} = - 68 ° (c = 1,00 ; DMSO)

### Exemple 17

### 5-chloro-3-pyridinyl 2,3,4-tri-O-acétyl-5-thio-β-D-xylopyranoside

En opérant de façon analogue à l'exemple 1, au départ du 5-chloro-3-pyridinol, on obtient le produit attendu sous forme d'une poudre blanche (rendement = 29 %). F=160°C
[α]²⁴_{D}= - 73 ° (c = 0,48 ; CHCl₃)

### Exemple 18

### 5-chloro-3-pyridinyl 5-thio-β-D-xylopyranoside

En opérant de façon analogue à l'exemple 1, au départ du composé obtenu selon l'exemple 17, on obtient le produit attendu sous forme d'une poudre blanche (rendement = 90 %).
F=184°C
[α]²⁴_{D}= - 82 ° (c = 0,47 ; DMSO)

### Exemple 19

### 2-cyano-3-pyridinyl 2,3,4-tri-O-acétyl-5-thio-β-D-xylopyranoside

On prépare une solution de 1,7 g (14,17 mM) de 2-cyano-3-pyridinol dans 30 ml d'acétonitrile et on ajoute, à l'abri de la lumière, 4,3 g (18,3 mM) d'oxyde d'argent et 3 g de tamis moléculaire 13X. Le mélange est agité pendant 10 mn à 50 °C puis on ajoute 6,5 g (18,3 mM) de bromure de 2,3,4-tri-O-acétyl-β-D-xylopyranoside et on maintient le mélange réactionnel pendant 18 heures sous agitation à 50 °C. Le mélange est ensuite refroidi à température ambiante et filtré sur adjuvant de filtration. Le filtrat est dilué avec de l'acétate d'éthyle et lavé avec de l'eau, une solution N de soude puis à l'eau jusqu'à pH neutre, et enfin séché sur sulfate de magnésium et concentré sous pression réduite. L'huile résiduelle est cristallisée par addition d'éther éthylique. On obtient 0,89 g du produit attendu sous forme de cristaux beiges (rendement = 16 %).
RMN ¹H (300 MHz ; CDCl₃) δ : 8,43 (m, 1H) ; 7,53 (m, 2H) ; 5,49 (t, 1H) ; 5,30 (d, 1H) ; 5,19 (m, 2H) ; 3,18 (m, 1H) ; 2,76 (m, 1H) ; 2,10 (m, 9H).
Le produit contient une faible proportion de dérivés α, dont le proton anomère donne des signaux à δ = 5,76 et δ = 5,63).

### Exemple 20

### 2-cyano-3-pyridinyl 5-thio-β-D-xylopyranoside

En opérant de façon analogue à l'exemple 2, au départ du composé obtenu selon l'exemple 19, on obtient le produit attendu sous forme de cristaux blancs (rendement = 60 %).
F=174°C
[α]²³_{D}= - 43 ° (c = 0,30 ; DMSO)

### Exemple 21

### 6-méthyl-3-pyridinyl 2,3,4-tri-O-acétyl-5-thio-β-D-xylopyranoside

En opérant de façon analogue à l'exemple 11, au départ de 6-méthyl-3-pyridinol, on obtient le produit brut qui est ensuite purifié par chromatographie sur gel de silice en éluant à l'aide d'un mélange dichlorométhane/éther éthylique (1/1 ; v/v), puis recristallisation dans l'acétate d'éthyle. On obtient ainsi le produit attendu sous forme d'un solide jaune (rendement = 20 %).
F = 130 °C
[α]²²_{D} = - 65 ° (c = 0,17 ; CHCl₃)

### Exemple 22

### 6-méthyl-3-pyridinyl 5-thio-β-D-xylopyranoside

En opérant de façon analogue à l'exemple 2, au départ du composé obtenu selon l'exemple 21, on obtient le produit attendu sous forme de cristaux blancs (rendement = 80 %).
F = 233°C
[α]²²_{D} = - 90 ° (c = 0,155 ; CH₃OH)

### Exemple 23

### Acide 5-[(2,3,4-tri-O-acétyl-5-thio-β-D-xylopyranosyl)oxy]-3-pyridinecarboxylique, méthyl ester

En opérant de façon analogue à l'exemple 1, au départ du 5-hydroxy-3-pyridinecarboxylate de méthyle, on obtient le produit attendu sous forme d'un solide beige (rendement = 7 %).
F = 50 °C
[α]²⁴_{D}= - 8 ° (c = 0,3 ; CH₃OH)

### Exemple 24

### Acide 5-[(5-thio-β-D-xylopyranosyl)oxy]-3-pyridinecarboxylique, méthyl ester

En opérant de façon analogue à l'exemple 2, au départ du composé obtenu selon l'exemple 23, on obtient le produit attendu sous forme d'une poudre fine beige (rendement = 99 %).
F = 137 °C
[α]²⁴_{D} = - 48 ° (c = 0,3 ; CH₃OH)

### Exemple 25

### 2-bromo-3-pyridinyl 2,3,4-tri-O-acétyl-5-thio-β-D-xylopyranoside

En opérant de façon analogue à l'exemple 1, au départ de 2-bromo-3-pyridinol, on obtient le produit attendu sous forme d'une poudre blanche (rendement = 40 %). F = 161 °C
[α]²²_{D}= - 66 ° (c = 1,8 ; CHCl₃)

### Exemple 26

### 2-bromo-3-pyridinyl 5-thio-β-D-xylopyranoside

En opérant de façon analogue à l'exemple 2, au départ du composé obtenu selon l'exemple 25, on obtient le produit attendu sous forme d'une poudre blanche (rendement = 85 %).
F = 151 °C
[α]²²_{D} = - 51 ° (c = 0,16 ; DMSO)

### Exemple 27

### 2-nitro-3-pyridinyl 2,3,4-tri-O-acétyl-5-thio-β-D-xylopyranoside

En opérant de façon analogue à l'exemple 19, au départ de 2-nitro-3-pyridinol, on obtient le produit attendu sous forme d'une poudre blanc cassé (rendement = 28 %).
F =178 °C
[α]²²_{D} = - 132 ° (c = 0,25 ; CHCl₃)

### Exemple 28

### 6-méthyl-2-nitro-3-pyridinyl 2,3,4-tri-O-acétyl-5-thio-β-D-xylopyranoside

En opérant de façon analogue à l'exemple 1, au départ de 6-méthyl-2-nitro-3-pyridinol, on obtient le produit attendu sous forme d'une poudre blanche (rendement = 11 %).
F = 154 °C
[α]²¹_{D}= - 92 ° (c = 0,48; CHCl₃)

### Exemple 29

### 3-pyridinyl 2,3,4-tris-O-(2-méthylpropanoyl)-5-thio-β-D-xylopyranoside

On prépare une solution de 0,6 g (2,4 mM) du composé obtenu selon l'exemple 2 dans 25 ml de pyridine et on ajoute 30 mg (0,24 mM) de 4-(diméthylamino)pyridine. On ajoute ensuite sous agitation et à température ambiante, 1,18 g (11 mM) de chlorure de 2-méthylpropanoyle. Le mélange réactionnel est agité pendant 1 heure à 60 °C, puis dilué avec 60 ml de toluène et concentré sous pression réduite. Le résidu d'évaporation est purifié par chromatographie sur gel de silice en éluant à l'aide d'un mélange toluène/acétate d'éthyle (75/25 ; v/v). On obtient ainsi 810 mg du composé attendu (rendement = 73 %).
RMN ¹H (300 MHz ; DMSO) δ : 8,33 (m, 1H) ; 8,26 (m, 1H) ; 7,50 (m, 1H) ; 7,40 (m, 1H) ; 5,96 (d, 1H) ; 5,34 (m, 2H) ; 5,01 (m, 1H) ; 3,04 (m, 1H) ; 2,88 (m, 1H) ; 2,42 (m, 3H) ; 1,02 (m, 12H) ; 0,94 (t, 6H).

### Exemple 29A

### 3-pyridinyl 2,3,4-tris-O-(2-méthylpropanoyl)-5-thio-β-D-xylopyranoside, méthanesulfonate

On prépare une solution de 802 mg (1,76 mM) du composé obtenu selon l'exemple 29 dans 5 ml d'acétate d'éthyle et on ajoute 2 ml d'éther éthylique, puis une solution de 131 µl (1,78 mM) d'acide méthanesulfonique dans 4 ml de tétrahydrofurane. Il se forme un précipité blanc. Le mélange réactionnel est agité 15 mn puis refroidi à 5 °C et filtré. On obtient ainsi 768 mg du sel attendu sous forme d'une poudre blanche (rendement = 81 %).
F = 215 °C
[α]²⁴_{D} = - 70 ° (c = 0,44; CH₃OH)

### Exemple 30

### 3-pyridinyl 2,3,4-tris-O-(3-méthylbutanoyl)-5-thio-β-D-xylopyranoside

En opérant de façon analogue à l'exemple 29, au départ du chlorure de 3-méthylbutanoyle, on obtient le produit attendu sous forme d'un solide blanc (rendement = 53 %).
RMN ¹H (300 MHz ; DMSO) δ : 8,52 (m, 1H) ; 8,41 (m, 1H) ; 7,83 (m, 1H) ; 7,65 (m, 1H) ; 6,00 (d, 1H) ; 5,40 (t, 1H) ; 5,30 (t, 1H) ; 5,00 (m, 1H) ; . 3,00 (m, 2H) ; 2,12 (m, 6H) ; 1,95 (m, 3H) ; 0,82 (m, 18H).

### Exemple 30A

### 3-pyridinyl 2,3,4-tris-O-(3-méthylbutanoyl)-5-thio-β-D-xylopyranoside, méthanesulfonate

En opérant de façon analogue à l'exemple 29A, au départ du composé obtenu selon l'exemple 30, on obtient le produit attendu sous forme d'une poudre blanche (rendement = 59 %).
F = 164 °C
[α]²⁴_{D}= - 68 ° (c = 0,28; CH₃OH)

### Exemple 31

### 3-pyridinyl 2,3,4-tris-O-(méthoxycarbonyl)-5-thio-β-D-xylopyranoside

On prépare une suspension de 0,3 g (1,23 mM) du composé obtenu selon l'exemple 2 dans 10 ml d'acétonitrile et 10 ml de tétrahydrofurane et on ajoute environ 30 mg de 4-(diméthylamino)pyridine et 300 mg de tamis moléculaire 4Å. On ajoute ensuite 0,8 ml (7,5 mM) de pyrocarbonate de diméthyle. Le mélange est agité pendant 24 heures à température ambiante et filtré. Le filtrat est concentré sous pression réduite et le résidu est purifié par chromatographie sur gel de silice en éluant à l'aide d'un mélange acétate d'éthyle/hexane (7/3 ; v/v). On obtient ainsi 0,25 g du composé attendu sous forme d'une poudre blanche (rendement = 50 %).
F=62 °C
[α]²³_{D} = - 78 ° (c = 0,15; CHCl₃)

### Exemple 31A

### 3-pyridinyl 2,3,4-tris-O-(méthoxycarbonyl)-5-thio-β-D-xylopyranoside, chlorhydrate

On prépare une solution de 560 mg (1,34 mM) du composé obtenu selon l'exemple 31 dans 150 ml d'éther éthylique anhydre et on ajoute 0,56 ml d'une solution de chlorure d'hydrogène dans l'éther éthylique (solution 2,3 N). Le sel précipite sous forme d'un solide très fin, que l'on extrait avec de l'eau pure. La phase aqueuse séparée est lyophilisée. On obtient ainsi 550 mg du composé attendu sous forme d'une poudre blanche (rendement = 90 %).
F = 155 °C
[α]²²_{D}= - 34 ° (c = 0,55; DMSO)

### Exemple 31B

### 3-pyridinyl 2,3,4-tris-O-(méthoxycarbonyl)-5-thio-β-D-xylopyranoside, méthanesulfonate

On prépare une solution de 200 mg (0,48 mM) du composé obtenu selon l'exemple 31 et on ajoute 50 mg (0,52 mM) d'une solution d'acide méthanesulfonique dans 4 ml d'éther éthylique et 0,2 ml de méthanol. Le précipité formé est séparé par filtration, repris en solution dans l'eau et la solution est lyophilisée. On obtient ainsi 230 mg du sel attendu sous forme d'un fin solide blanc (rendement = 96 %).
F = 148°C
[α]²⁵_{D}= - 68 ° (c =0,18 CH₃OH)

### Exemple 32

### Chlorure de 1-[[[(diméthylamino)carbonyl]oxy]methyl]-3-[(5-thio-β-D-xylopyranosyl)oxy]pyridinium

On mélange 1 g (4,1 mM) du composé obtenu selon l'exemple 2 dans 100 ml d'acétonitrile. On ajoute 0,62 g (4,1 mM) d'iodure de sodium et 0,68 g (4,9 mM) de chlorométhyl(diméthylamino)formate. Le mélange réactionnel est agité pendant 2 heures à température ambiante, puis filtré. Le filtre est rincé avec 20 ml de dichlorométhane et 20 ml de méthanol et les filtrats rassemblés sont percolés sur une colonne de résine Dowex 1X8-200 Cl⁻, puis concentrés sous pression réduite. Le résidu d'évaporation est trituré dans l'acétone. Le solide formé est séparé par filtration, repris en solution dans l'eau et lyophilisé. On obtient ainsi 800 mg du composé attendu sous forme d'un solide fin beige (rendement = 51 %). F=117°C
[α]²⁴_{D} = - 77 ° (c = 0,22 CH₃OH)

### Exemple 33

### 3-pyridinyl 2,3,4-tris-O-(2,2-diméthylpropanoyl)-5-thio-β-D-xylopyranoside, pivalate

On prépare une solution de 1 g (4,11 mM) du composé obtenu selon l'exemple 2 dans 20 ml de pyridine et on ajoute 1 g de tamis moléculaire 4 Å, puis 7,6 ml (37 mM) d'anhydride triméthylacétique et 30 mg (0,24 mM) de 4-(diméthylamino)pyridine. Le mélange réactionnel est chauffé à reflux pendant 2 heures, puis agité à température ambiante pendant 24 heures. Le mélange est ensuite filtré, le filtre est rincé avec 20 ml de méthanol et les filtrats sont concentrés sous pression réduite. Le produit brut obtenu est purifié par chromatographie sur gel de silice en éluant à l'aide d'un mélange cyclohexane/acétate d'éthyle (55/45 ;v/v). On obtient ainsi 600 mg du triester attendu sous forme du sel avec l'acide triméthylacétique (pivalate). Rendement = 24 % (poudre blanche).
F=140°C
[α]²⁵_{D} = - 56 ° (c = 0,50; CH₃OH)

### Exemple 34

### 6-méthyl-2-pyridinyl 2,3,4-tri-O-acétyl-5-thio-β-D-xylopyranoside

En opérant de façon analogue à l'exemple 1, au départ de 6-méthyl-2-pyridinol, on obtient le produit attendu sous forme d'une poudre blanche (rendement = 10 %).
F=106°C
[α]²²_{D} = - 50 ° (c = 0,25; CHCl₃)

### Exemple 35

### 6-méthyl-2-pyridinyl 5-thio-β-D-xylopyranoside

En opérant de façon analogue à l'exemple 2, au départ du composé obtenu selon l'exemple 34, on obtient le produit attendu après lyophilisation sous forme d'une poudre blanche (rendement = 90 %).
F = 54 °C
[α]²⁵_{D}= - 63 ° (c = 0,26 ; DMSO)

### Exemple 36

### 2-chloro-3-pyridinyl 2,3,4-tri-O-acétyl-5-thio-β-D-xylopyranoside

En opérant de façon analogue à l'exemple 1, au départ de 2-chloro-3-pyridinol, on obtient le produit attendu sous forme d'un solide blanc (rendement = 37 %).
F = 160 °C
[α]²¹_{D}= - 108 ° (c = 0,43 ; CH₂Cl₂)

### Exemple 37

### 2-chloro-3-pyridinyl 5-thio-β-D-xylopyranoside

En opérant de façon analogue à l'exemple 8, au départ du composé obtenu selon l'exemple 36, on obtient le produit attendu sous forme d'un solide blanc (rendement = 97 %).
F = 153 °C
[α]²³_{D} = - 44 ° (c = 0,44 ; DMSO)

### Exemple 38

### 2-(diméthylaminométhyl)-3-pyridinyl 2,3,4-tri-O-acétyl-5-thio-β-D-xylopyranoside

En opérant de façon analogue à l'exemple 11, au départ de 2-(diméthylaminométhyl)-3-pyridinol, on obtient le produit attendu sous forme d'un solide jaune (rendement = 12 %).
F = 105 °C
[α]²¹_{D} =- 74 ° (c = 0,41 ; CH₂Cl₂)

### Exemple 39

### 2-(diméthylaminométhyl)-3-pyridinyl 5-thio-β-D-xylopyranoside

En opérant de façon analogue à l'exemple 8, au départ du composé obtenu selon l'exemple 38, on obtient le produit attendu sous forme d'un solide blanc (rendement = 90 %).
F = 79 °C
[α]²³_{D} =- 48 ° (c = 0,40 ; DMSO)

### Exemple 40

### 6-(trifluorométhyl)-3-pyridinyl 2,3,4-tri-O-acétyl-5-thio-β-D-xylopyranoside

En opérant de façon analogue à l'exemple 1, au départ de 6-(trifluorométhyl) -3-pyridinol, on obtient le produit attendu sous forme d'une poudre blanche (rendement = 28 %).
F = 156 °C
[α]³⁰_{D} = - 6 ° (c = 0,69 ; CHCl₃)

### Exemple 41

### 6-(trifluorométhyl)-3-pyridinyl 5-thio-β-D-xylopyranoside

En opérant de façon analogue à l'exemple 8, au départ du composé obtenu selon l'exemple 40, on obtient le produit attendu sous forme d'un solide beige (rendement = 97 %).
F = 170-175 °C
[α]²³_{D} = - 78 ° (c = 0,40 ; DMSO)

### Exemple 42

### 5-chloro-2-cyano-3-pyridinyl 2,3,4-tri-O-acétyl-5-thio-β-D-xylopyranoside

En opérant de façon analogue à l'exemple 1, au départ de 5-chloro-2-cyano-3-pyridinol, on obtient le produit attendu sous forme d'un solide blanc (rendement = 11 %).
F = 171 °C
[α]²³_{D}= - 137 ° (c = 0,17 ; CH₂Cl₂)

### Exemple 43

### 5-chloro-2-cyano-3-pyridinyl 5-thio-β-D-xylopyranoside

En opérant de façon analogue à l'exemple 8, au départ du composé obtenu selon l'exemple 42, on obtient le produit attendu sous forme d'un solide blanc cassé (rendement = 52 %).
F = 122 °C
[α]²⁰_{D} = - 71 ° (c = 0,28 ; CH₃OH)

### Exemple 44

### 4-cyano-2-méthyl-3-pyridinyl 2,3,4-tri-O-acétyl-5-thio-β-D-xylopyranoside

En opérant de façon analogue à l'exemple 5, au départ de 4-cyano-2-méthyl-3-pyridinol, on obtient le produit attendu sous forme d'un solide marron (rendement = 59 %).
F = 109-113 °C
[α]²³_{D} = - 4,8 ° (c = 0,50; CH₂Cl₂)

### Exemple 45

### 4-cyano-2-méthyl-3-pyridinyl 5-thio-β-D-xylopyranoside

En opérant de façon analogue à l'exemple 8, au départ du composé obtenu selon l'exemple 44, on obtient le produit attendu sous forme d'un solide rose pâle (rendement = 45 %).
F = 161 °C
[α]²³_{D}= - 2,5 ° (c = 0,50; CH₃OH)

### Exemple 46

### 2-fluoro-6-méthyl-3-pyridinyl 2,3,4-tri-O-acétyl-5-thio-β-D-xylopyranoside

En opérant de façon analogue à l'exemple 1, au départ de 2-fluoro-6-méthyl-3-pyridinol, on obtient le produit attendu sous forme d'une poudre blanche (rendement = 7%).
F=139°C
[α]²⁸_{D}= - 98 ° (c = 0,25 ; CHCl₃)

### Exemple 47

### 2-fluoro-6-méthyl-3-pyridinyl 5-thio-β-D-xylopyranoside

En opérant de façon analogue à l'exemple 2, au départ du composé obtenu selon l'exemple 46, on obtient le produit attendu sous forme d'une poudre blanche (rendement = 79 %).
F = 167-170 °C
[α]³¹ _{D} = - 85 ° (c = 0,22 ; DMSO)

### Exemple 48

### 5-fluoro-3-pyridinyl 2,3,4-tri-O-acétyl-5-thio-β-D-xylopyranoside

En opérant de façon analogue à l'exemple 1, au départ de 5-fluoro-3-pyridinol, on obtient le produit attendu sous forme d'un solide blanc (rendement = 20 %). F=143°C
[α]²⁵_{D}= - 78 ° (c = 0,51; CHCl₃)

### Exemple 49

### 5-fluoro-3-pyridinyl 5-thio-β-D-xylopyranoside

En opérant de façon analogue à l'exemple 8, au départ du composé obtenu selon l'exemple 48, on obtient le produit attendu sous forme d'une poudre blanche (rendement = 85 %).
F = 182-183 °C
[α]²⁵D= - 95 ° (c = 0,51 ; CH₃OH)

### Exemple 50

### 5-cyano-3-pyridinyl 2,3,4-tri-O-acétyl-5-thio-β-D-xylopyranoside

En opérant de façon analogue à l'exemple 1, au départ de 5-cyano-3-pyridinol, on obtient le produit attendu sous forme d'un solide blanc (rendement = 24 %).
F = 147 °C
[α]²⁰_{D}= + 3,6 ° (c = 0,34 ; CH₂Cl₂)

### Exemple 51

### 5-cyano-3-pyridinyl 5-thio-β-D-xylopyranoside

En opérant de façon analogue à l'exemple 8, au départ du composé obtenu selon l'exemple 50, on obtient le produit attendu sous forme d'un solide blanc (rendement = 52 %).
F = 149 °C
[α]²⁰_{D}= + 3 ° (c = 0,10 ; CH₃OH)

### Exemple 52

### 6-chloro-3-pyridiny 2,3,4-tri-O-acétyl-5-thio-β-D-xylopyranoside

En opérant de façon analogue à l'exemple 1, au départ de 6-chloro-3-pyridinol, on obtient le produit attendu sous forme d'un solide blanc (rendement = 18 %). F = 141 °C
[α]²³_{D}= - 64 ° (c = 0,50 ; CH₂Cl₂)

### Exemple 53

### 6-chloro-3-pyridinyl 5-thio-β-D-xylopyranoside

En opérant de façon analogue à l'exemple 8, au départ du composé obtenu selon l'exemple 52, on obtient le produit attendu sous forme d'un solide blanc (rendement = 89 %).
F = 218 °C
[α]²³D = - 70 ° (c = 0,50 ; DMSO)

### Exemple 54

### 4-cyano-3-pyridinyl 2,3,4-tri-O-acétyl-5-thio-β-D-xylopyranoside

En opérant de façon analogue à l'exemple 1, au départ de 4-cyano-3-pyridinol, on obtient le produit attendu sous forme d'un solide blanc (rendement = 7 %). F = 167 °C
[α]²³_{D} = - 148 ° (c = 0,15 ; CHCl₃)

### Exemple 55

### 4-cyano-3-pyridinyl 5-thio-β-D-xylopyranoside

En opérant de façon analogue à l'exemple 8, au départ du composé obtenu selon l'exemple 54, on obtient le produit attendu sous forme d'un solide blanc (rendement = 60 %).
F = 157 °C

### [α]²³_{D} 35° (c = 0,08 ; DMSO)

### Exemple 56

### 5-méthyl-3-pyridinyl 2,3,4-tri-O-acétyl-5-thio-β-D-xylopyranoside

En opérant de façon analogue à l'exemple 1, au départ de 5-méthyl-3-pyridinol, on obtient le produit attendu sous forme de cristaux couleur crème (rendement = 11%).
F = 134 °C
[α]²⁴_{D} = - 35 ° (c = 0,16 ; DMSO)

### Exemple 57

### 5-méthyl-3-pyridinyl 5-thio-β-D-xylopyranoside

En opérant de façon analogue à l'exemple 8, au départ du composé obtenu selon l'exemple 56, on obtient le produit attendu sous forme d'une poudre blanche (rendement = 72 %).
F = 213 °C
[α]²⁵_{D}= - 106 ° (c = 0,20 ; DMSO)

### Exemple 58

### 2-chloro-5-fluoro-3-pyridinyl 2,3,4-tri-O-acétyl-5-thio-β-D-xylopyranoside

En opérant de façon analogue à l'exemple 1, au départ de 2-chloro-5-fluoro-3-pyridinol, on obtient le produit attendu sous forme d'une poudre blanche (rendement = 11 %).
F = 185 °C
[α]²⁷_{D} = - 93 ° (c = 0,11 ; DMSO)

### Exemple 59

### 2-chloro-5-fluoro-3-pyridinyl 5-thio-β-D-xylopyranoside

En opérant de façon analogue à l'exemple 2, au départ du composé obtenu selon l'exemple 58, on obtient le produit attendu sous forme d'une poudre blanche (rendement = 65 %).
F = 143 °C
[α]³⁰_{D} = - 79 ° (c = 0,18 ; DMSO)

### Exemple 60

### 6-fluoro-3-pyridinyl 2,3,4-tri-O-acétyl-β-thio-β-D-xylopyranoside

En opérant de façon analogue à l'exemple 1, au départ de 6-fluoro-3-pyridinol, on obtient le produit attendu sous forme de cristaux blancs (rendement = 5 %).
F = 123°C
[α]²⁸_{D}= - 39 ° (c = 0,17 ; DMSO)

### Exemple 61

### 6-fluoro-3-pyridinyl 5-thio-β-D-xylopyranoside

En opérant de façon analogue à l'exemple 8, au départ du composé obtenu selon l'exemple 60, on obtient le produit attendu sous forme d'un solide léger blanc (rendement = 74 %).
F = 209-210 °C
[α]²⁷_{D} = - 119 ° (c = 0,14 ; DMSO)

### Exemple 62

### N-[5-[( 2,3,4-tri-O-acétyl-5-thio-β-D-xylopyranosyl)oxy]-2-pyridinyl] acétamide

En opérant de façon analogue à l'exemple 1, au départ de N-(5-hydroxy-2-pyridinyl)acétamide, on obtient le produit attendu sous forme d'une poudre blanche (rendement = 11 %).
F = 132-133 °C
[α]³²_{D}= - 1,6 ° (c = 0,20 ; DMSO)

### Exemple 63

### N-[5-[(5-thio-β-D-xylopyranosyl)oxy]-2-pyridinyl]acétamide

En opérant de façon analogue à l'exemple 2, au départ du composé obtenu selon l'exemple 62, on obtient le produit attendu sous forme d'une poudre blanche (rendement = 31 %).
F = 209°C
[α]²⁵D = - 64° (c = 0,40; DMSO)

### Exemple 64

### N-[5-[( 2,3,4-tri-O-acétyl-5-thio-β-D-xylopyranosyl)oxyl-2-pyridinyl] méthanesulfonamide

En opérant de façon analogue à l'exemple 1, au départ de N-(5-hydroxy-2-pyridinyl)méthanesulfonamide, on obtient le produit attendu sous forme de paillettes blanches (rendement = 12 %).
F = 185-190 °C
[α]²⁸_{D} = - 5,5° (c = 0,30 ; DMSO)

### Exemple 65

### N-[5-[(5-thio-β-D-xylopyranosyl)oxy]-2-pyridinyl]méthanesulfonamide

En opérant de façon analogue à l'exemple 8, au départ du composé obtenu selon l'exemple 64, on obtient le produit attendu sous forme d'une poudre blanche (rendement = 83 %).
F = 201-204 °C
[α]²⁷_{D}= - 48° (c = 0,30; DMSO)

### Exemple 66

### 4-(trifluorométhyl)-3-pyridinyl 2,3,4-tri-O-acétyl-5-thio-β-D-xylopyranoside

En opérant de façon analogue à l'exemple 1, au départ de 4-(trifluorométhyl)-3-pyridinol, on obtient le produit attendu sous forme d'une poudre blanche (rendement = 9 %).
F = 198°C
[α]³²_{D} = - 72 ° (c = 0,27 ; DMSO)

### Exemple 67

### 4-(trifluorométhyl)-3-pyridinyl 5-thio-β-D-xylopyranoside

En opérant de façon analogue à l'exemple 2, au départ du composé obtenu selon l'exemple 66, on obtient le produit attendu sous forme d'aiguilles blanches (rendement = 60 %).
F = 191 °C
[α]³²_{D} =- 34 ° (c = 0,30 ; DMSO)

### Exemple 68

### 2-fluoro-3-pyridinyl 2,3,4-tri-O-acétyl-5-thio-β-D-xylopyranoside

En opérant de façon analogue à l'exemple 11, au départ de 2-fluoro-3-pyridinol, on obtient le produit attendu sous forme d'une poudre blanche (rendement = 32 %).
F = 160°C
[α]²⁸_{D} = - 86 ° (c = 0,20 ; CHCl₃)

### Exemple 69

### 2-fluoro-3-pyridinyl 5-thio-β-D-xylopyranoside

En opérant de façon analogue à l'exemple 2, au départ du composé obtenu selon l'exemple 68, on obtient le produit attendu sous forme d'un solide léger blanc (rendement = 31 %).
F = 85-87 °C
[α]²⁸_{D} = - 40°(c = 0,30 ; CH₃OH)

### Exemple 70

### 2,6-diméthyl-3-pyridinyl 2,3,4-tri-O-acétyl-5-thio-β-D-xylopyranoside

En opérant de façon analogue à l'exemple 1, au départ de 2,6-diméthyl-3-pyridinol, on obtient le produit attendu sous forme d'une poudre blanche (rendement = 7 %).
F = 139-140°C
[α]²⁹_{D} = - 40 ° (c = 0,20 ; DMSO)

### Exemple 71

### 2,6-diméthyl-3-pyridinyl 5-thio-β-D-xylopyranoside

En opérant de façon analogue à l'exemple 8, au départ du composé obtenu selon l'exemple 70, on obtient le produit attendu sous forme d'une poudre blanche (rendement = 91 %).
F = 186°C
[α]²⁶_{D} = - 64 ° (c = 0,20 ; DMSO)

### Exemple 72

### 6-chloro-5-fluoro-3-pyridinyl 2,3,4-tri-O-acétyl-5-thio-β-D-xylopyranoside

En opérant de façon analogue à l'exemple 1, au départ de 6-chloro-5-fluoro-3-pyridinol, on obtient le produit attendu sous forme de paillettes blanches (rendement = 43%).
F = 158-162°C
[α]²⁷_{D} = - 21 ° (c = 0,30 ; DMSO)

### Exemple 73

### 6-chloro-5-fluoro-3-pyridinyl 5-thio-β-D-xylopyranoside

En opérant de façon analogue à l'exemple 8, au départ du composé obtenu selon l'exemple 72, on obtient le produit attendu sous forme d'aiguilles blanches (rendement = 26 %).
F = 198-200 °C
[α]²⁷_{D} = - 68 ° (c = 0,10 ; DMSO)

### Exemple 74

### 6-méthoxy-3-pyridinyl 5-thio-β-D-xylopyranoside

On prépare un mélange de 100 mg (0,8 mM) de 6-méthoxy-3-pyridinol, 568 mg (1,6 mM) de bromure de 2,3,4-tri-O-acétyl-5-thio-α-D-xylopyranosyle, 50 mg (0,16 mM) de chlorure de benzyl-tributylammonium dans 2 ml de chloroforme et on ajoute 552 mg (4 mM) de carbonate de potassium, puis 30 µl d'eau. Le mélange réactionnel est agité pendant 3 heures à 50 °C, puis une nuit à température ambiante. On ajoute 8ml de chloroforme et la phase organique obtenue est lavée à l'eau et avec une solution de bicarbonate de sodium, puis séchée sur sulfate de magnésium et concentrée sous pression réduite. Le résidu d'évaporation est repris dans 8 ml de méthanol à 50°C et filtré. Le filtrat est concentré sous pression réduite et le produit brut est purifié par chromatographie sur gel de silice en éluant à l'aide d'un mélange cyclohexane/acétate d'éthyle (8/2 ;v/v). On obtient ainsi 100 mg d'une huile jaune qui est à nouveau purifiée par chromatographie sur gel de silice en éluant avec un mélange dichlorométhane/méthanol (95/5 ;v/v) pour donner 71 mg du composé attendu sous forme d'une poudre blanche (rendement = 22 %)
F = 159-161 °C
[α]²⁴_{D}= - 98 ° (c = 0,10 ; DMSO)

### Exemple 75

### 6-cyano-5-fluoro-3-pyridinyl 2,3,4-tri-O-acétyl-5-thio-β-D-xylopyranoside

En opérant de façon analogue à l'exemple 5, au départ de 6-cyano-5-fluoro-3-pyridinol, on obtient le produit attendu sous forme d'une poudre blanche (rendement = 38%).
F = 176-177 °C
[α]²⁹_{D} = - 37 ° (c = 0,37 ; DMSO)

### Exemple 76

### 6-cyano-5-fluoro-3-pyridinyl 5-thio-β-D-xylopyranoside

En opérant de façon analogue à l'exemple 2, au départ du composé obtenu selon l'exemple 75, on obtient le produit attendu sous forme d'une poudre blanche (rendement = 64 %).
F = 155-156 °C
[α]²⁹_{D} = - 72 ° (c = 0,44 ; DMSO)

### Exemple 77

### 6-cyano-2-méthyl-3-pyridinyl 2,3,4-tri-O-acétyl-5-thio-β-D-xylopyranoside

En opérant de façon analogue à l'exemple 1, au départ de 6-cyano-2-méthyl-3-pyridinol, on obtient le produit attendu sous forme d'un solide blanc (rendement = 27%).
F = 128-131 °C
[α]³⁰_{D} = - 58 ° (c = 0,21 ; DMSO)

### Exemple 78

### 6-cyano-2-méthyl-3-pyridinyl 5-thio-β-D-xylopyranoside

En opérant de façon analogue à l'exemple 2, au départ du composé obtenu selon l'exemple 77, on obtient le produit attendu sous forme d'aiguilles blanches (rendement = 47 %).
F = 193-195 °C
[α]²⁵_{D} = - 80° (c = 0,19 ; DMSO)

### Exemple 79

### 2-cyano-5-fluoro-3-pyridinyl 2,3,4-tri-O-acétyl-5-thio-β-D-xylopyranoside

En opérant de façon analogue à l'exemple 5, au départ de 2-cyano-5-fluoro-3-pyridinol, on obtient le produit attendu sous forme d'un solide blanc (rendement = 17%).
F = 150°C
[α]³⁰_{D} = - 75 ° (c = 0,30 ; DMSO)

### Exemple 80

### 2-cyano-5-fluoro-3-pyridinyl 5-thio-β-D-xylopyranoside

En opérant de façon analogue à l'exemple 2, au départ du composé obtenu selon l'exemple 79, on obtient le produit attendu sous forme d'un solide blanc (rendement = 57 %).
F = 94°C
[α]²⁷_{D} = - 27 ° (c = 0,30 ; DMSO)

### Exemple 81

### 4-méthyl-3-pyridinyl 2,3,4-tri-O-acétyl-5-thio-β-D-xylopyranoside

En opérant de façon analogue à l'exemple 1, au départ de 4-méthyl-3-pyridinol, on obtient le produit attendu sous forme d'un solide beige (rendement = 14%). F = 150°C
[α]³⁰_{D} = - 65 ° (c = 0,21 ; DMSO)

### Exemple 82

### 4-méthyl-3-pyridinyl 5-thio-β-D-xylopyranoside

En opérant de façon analogue à l'exemple 2, au départ du composé obtenu selon l'exemple 81, on obtient le produit attendu sous forme d'un solide blanc (rendement = 90 %).
F = 236°C
[α]³¹_{D} = - 88 ° (c = 0,20; DMSO)

### Exemple 83

### 6-cyano-3-pyridinyl 2,3,4-tri-O-acétyl-5-thio-β-D-xylopyranoside

En opérant de façon analogue à l'exemple 1, au départ de 6-cyano-3-pyridinol, on obtient le produit attendu sous forme d'une poudre blanche (rendement = 24%).
F = 147 °C
[α]²⁵_{D} = - 27 ° (c = 0,28; DMSO)

### Exemple 84

### 6-cyano-3-pyridinyl 5-thio-β-D-xylopyranoside

En opérant de façon analogue à l'exemple 2, au départ du composé obtenu selon l'exemple 83, on obtient le produit attendu sous forme d'un solide blanc (rendement = 77 %).
F = 179-180 °C
[α]³⁰_{D} = - 77 ° (c = 0,25 ; DMSO)

### Exemple 85

### 5-(trifluorométhyl)-3-pyridinyl 2,3,4-tri-O-acétyl-5-thio-β-D-xylopyranoside

En opérant de façon analogue à l'exemple 1, au départ de 5-(trifluorométhyl)-3-pyridinol, on obtient le produit attendu sous forme de cristaux blancs (rendement = 31%).
F = 111-112°C
[α]²⁸_{D} = - 37° (c = 0,30 ; DMSO)

### Exemple 86

### 5-(trifluorométhyl)-3-pyridinyl 5-thio-β-D-xylopyranoside

En opérant de façon analogue à l'exemple 8, au départ du composé obtenu selon l'exemple 85, on obtient le produit attendu sous forme d'un solide floconneux blanc (rendement = 88%).
F = 160-161 °C
[α]²⁵_{D} = - 79 ° (c = 0,32; DMSO)

### Exemple 87

### 3-pyridinyl 2,3,4-tris-O-(éthoxycarbonyl)-5-thio-β-D-xylopyranoside

On prépare une suspension de 500 mg (2,05 mM) du composé obtenu selon l'exemple 2 dans 5 ml de tétrahydrofurane et 5 ml d'acétonitrile et on ajoute 25 mg (2,5 mM) de 4-(diméthylamino)pyridine. On ajoute ensuite progressivement sous agitation et à température ambiante, 1,83 ml (12,3 mM) de pyrocarbonate de diéthyle ; Le mélange réactionnel est agité à température ambiante pendant 7 heures puis filtré et le filtrat est concentré sous pression réduite. Le produit brut est purifié par chromatographie sur gel de silice en éluant à l'aide d'un mélange toluène/acétate d'éthyle (7/3 ;v/v). On obtient ainsi 638 mg du composé attendu sous forme d'une huile incolore qui cristallise lentement (rendement = 67 %)
F = 41°C
[α]²²_{D} = - 55 ° (c = 0,31 ; CH₂Cl₂)

### Exemple 88

### 2-chloro-3-pyridinyl 2,3,4-tris-O-(éthoxycarbonyl)-5-thio-β-D-xylopyranoside

En opérant de façon analogue à l'exemple 87, au départ du composé obtenu selon l'exemple 37, on obtient le produit attendu sous forme d'un solide blanc (rendement = 87 %).
F = 49-52 °C
[α]²⁵_{D} = - 78 ° (c = 0,40 ; CHCl₃)

### Exemple 89

### 6-(trifluorométhyl)-3-pyridinyl 2,3,4-tris-O-(éthoxycarbonyl)~5-thio-β-D-xylopyranoside

En opérant de façon analogue à l'exemple 87, au départ du composé obtenu selon l'exemple 41, on obtient le produit attendu sous forme d'un solide blanc (rendement = 89 %).
F = 55-57°C
[α]²⁶_{D} = - 76 ° (c = 0,36 ; CHCl₃)

### Exemple 90

### 2-cyano-3-pyridinyl 2,3,4-tris-O-(éthoxycarbonyl)-5-thio-β-D-xylopyranoside

En opérant de façon analogue à l'exemple 87, au départ du composé obtenu selon l'exemple 20, on obtient le produit attendu sous forme d'un solide incolore (rendement = 81 %).
F = 44-48 °C
[α]²⁵_{D} = - 76° (c = 0,42; CHCl₃)

### Exemple 91

### 3-pyridinyl 2,3,4-tri-O-[(1-piperidinyl)acetyl]-5-thio-β-D-xylopyranoside

On prépare un mélange de 4,64 g (24,2 mM) de EDCI [1-(3-diméthylaminopropyl)-3-éthylcarbodiimide chlorhydrate] et 2,34 g (23,2 mM) de triéthylamine dans 100 ml de dichlorométhane, puis on ajoute sous agitation, 1,22 g (5,04 mM) du composé obtenu selon l'exemple 2, 3,46 g (24,2 mM) d'acide 1-pipéridineacétique, 3,33 g (24,7 mM) de HOBT (1-hydroxybenzotriazole) et 0,92 g de 4-(diméthylamino)pyridine. Le mélange réactionnel est agité à température ambiante pendant 20 heures puis filtré et le filtrat lavé à l'eau (2 fois), séché sur sulfate de magnésium et concentré sous pression réduite. Le produit brut est purifié par chromatographie sur gel de silice en éluant à l'aide d'un mélange dichlorométhane/méthanol (95/5 ;v/v) . On obtient ainsi une huile qui cristallise après trituration dans l'éther isopropylique pour donner 670 mg du composé attendu sous forme d'une poudre blanche (rendement = 21%)
F=116°C
[α]²⁸_{D} = - 21° (c = 0,17; DMSO)

### Exemple 92

### 3-pyridinyl 2,3,4-tri-O-propanoyl-5-thio-β-D-xylopyranoside

En opérant de façon analogue à l'exemple 29, au départ de chlorure de propanoyle, on obtient le produit attendu sous forme d'une poudre crème (rendement = 21%).
F=104°C
[α]²³_{D} = - 72° (c = 0,38 ; CHCl₃)

### Exemple 93

### 3-pyridinyl 2,3,4-tri-O-butanoyl-5-thio-β-D-xylopyranoside

En opérant de façon analogue à l'exemple 29, au départ de chlorure de butanoyle, on obtient le produit attendu sous forme d'une poudre blanche (rendement = 34%).
F = 70°C
[α]²⁷_{D} = - 26 ° (c = 0,15 ; DMSO)

Les structures des composés de formule I décrits ci-dessus sont reprises dans le tableau suivant (P indique la position du groupe D ou L-xylose sur le noyau pyridine) :

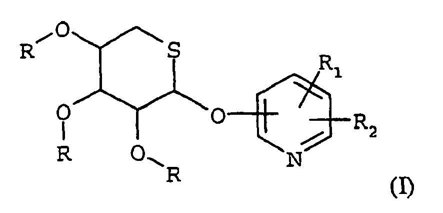

| Exemple | R1 | R2 | P | R | Sel |
|---|---|---|---|---|---|
| 1 | H | H | 3 (D) | Ac | - |
| 1A | H | H | 3 (D) | Ac | Ms |
| 2 | H | H | 3 (D) | H | - |
| 2A | H | H | 3 (D) | H | Ms |
| 2B | H | H | 3 (D) | H | HSulf |
| 2C | H | H | 3 (D) | H | Chlo |
| 2D | H | H | 3 (D) | H | Bromh |
| 3 | H | H | 3 (L) | Ac | - |
| 4 | H | H | 3 (L) | H | - |
| 5 | H | H | 2 (D) | Ac | - |
| 6 | H | H | 2 (D) | H | - |
| 7 | H | H | 3 (D) | Ac | N-Oxyde |
| 8 | H | H | 3 (D) | H | N-Oxyde |
| 9 | H | H | 4 (D) | Ac | - |
| 10 | H | H | 4 (D) | H | - |
| 11 | 2-CH₃ | H | 3 (D) | Ac | - |
| 12 | 2-CH₃ | H | 3 (D) | H | - |
| 13 | 2-Bn | H | 3 (D) | Ac | - |
| 14 | 2-Bn | H | 3 (D) | H | - |
| 15 | 2-C₂H₅ | 4-CH₃ | 3 (D) | Ac | - |
| 16 | 2-C₂H₅ | 4-CH₃ | 3 (D) | H | - |
| 17 | 5-Cl | H | 3 (D) | Ac | - |
| 18 | 5-Cl | H | 3 (D) | H | - |
| 19 | 2-CN | H | 3 (D) | Ac | - |
| 20 | 2-CN | H | 3 (D) | H | - |
| 21 | 6-CH₃ | H | 3 (D) | Ac | - |
| 22 | 6-CH₃ | H | 3 (D) | H | - |
| 23 | 5-COOCH₃ | H | 3 (D) | Ac | - |
| 24 | 5-COOCH₃ | H | 3 (D) | H | - |
| 25 | 2-Br | H | 3 (D) | Ac | |
| 26 | 2-Br | H | 3 (D) | H | - |
| 27 | 2-NO₂ | H | 3 (D) | Ac | - |
| 28 | 2-NO₂ | 6-CH3 | 3 (D) | Ac | - |
| 29 | H | H | 3 (D) | (CH₃)₂CHCO | - |
| 29A | H | H | 3 (D) | (CH₃)₂CHCO | Ms |
| 30 | H | H | 3 (D) | (CH₃)₂CH-CH2-CO | |
| 30A | H | H | 3 (D) | (CH₃)₂CH-CH2-CO | Ms |
| 31 | H | H | 3 (D) | COOCH₃ | |
| 31A | H | H | 3 (D) | COOCH₃ | Chlor |
| 31B | H | H | 3 (D) | COACH₃ | Ms |
| 32 | H | H | 3 (D) | H | Cl⁻ Quatern |
| 33 | H | H | 3(D) | CO-C(CH₃)₃ | piv |
| 34 | 6-CH₃ | H | 2(D) | Ac | - |
| 35 | 6-CH₃ | H | 2(D) | H | - |
| 36 | 2-Cl | H | 3(D) | Ac | - |
| 37 | 2-Cl | H | 3(D) | H | - |
| 38 | 2-CH₂-N(CH₃)₂ | H | 3(D) | Ac | - |
| 39 | 2-CH₂-N(CH₃)₂ | H | 3(D) | H | - |
| 40 | 6-CF3 | H | 3(D) | Ac | - |
| 41 | 6-CF3 | H | 3(D) | H | - |
| 42 | 5-Cl | 2-CN | 3(D) | Ac | - |
| 43 | 5-Cl | 2-CN | 3(D) | H | - |
| 44 | 4-CN | 2-CH₃ | 3(D) | Ac | - |
| 45 | 4-CN | 2-CH₃ | 3(D) | H | - |
| 46 | 2-F | 6-CH₃ | 3(D) | Ac | |
| 47 | 2-F | 6-CH₃ | 3(D) | H | |
| 48 | 5-F | H | 3(D) | Ac | - |
| 49 | 5-F | H | 3(D) | H | - |
| 50 | 5-CN | H | 3(D) | Ac | - |
| 51 | 5-CN | H | 3(D) | H | - |
| 52 | 6-Cl | H | 3(D) | Ac | - |
| 53 | 6-Cl | H | 3(D) | H | - |
| 54 | 4-CN | H | 3(D) | Ac | - |
| 55 | 4-CN | H | 3(D) | H | - |
| 56 | 5-CH₃ | H | 3(D) | Ac | - |
| 57 | 5-CH₃ | H | 3(D) | H | - |
| 58 | 2-Cl | 5-F | 3(D) | Ac | - |
| 59 | 2-Cl | 5-F | 3(D) | H | - |
| 60 | 6-F | H | 3(D) | Ac | - |
| 61 | 6-F | H | 3(D) | H | - |
| 62 | 6-NH-CO-CH₃ | H | 3(D) | Ac | - |
| 63 | 6-NH-CO-CH₃ | H | 3(D) | H | - |
| 64 | 6-NH-SO₂-CH₃ | H | 3(D) | Ac | - |
| 65 | 6-NH-SO₂-CH₃ | H | 3(D) | H | - |
| 66 | 4-CF3 | H | 3(D) | Ac | - |
| 67 | 4-CF3 | H | 3(D) | H | - |
| 68 | 2-F | H | 3(D) | Ac | - |
| 69 | 2-F | H | 3(D) | H | - |
| 70 | 2-CH₃ | 6-CH₃ | 3(D) | Ac | - |
| 71 | 2-CH₃ | 6-CH₃ | 3(D) | H | - |
| 72 | 5-F | 6-Cl | 3(D) | Ac | - |
| 73 | 5-F | 6-Cl | 3(D) | H | - |
| 74 | 6-OCH₃ | H | 3(D) | H | |
| 75 | 5-F | 6-CN | 3(D) | Ac | |
| 76 | 5-F | 6-CN | 3(D) | H | |
| 77 | 2-CH₃ | 6-CN | 3(D) | Ac | |
| 78 | 2-CH₃ | 6-CN | 3(D) | H | |
| 79 | 2-CN | 5-F | 3(D) | Ac | |
| 80 | 2-CN | 5-F | 3(D) | H | |
| S1 | 4-CH₃ | H | 3(D) | Ac | |
| 82 | 4-CH₃ | H | 3(D) | H | |
| 83 | 6-CN | H | 3(D) | Ac | |
| 84 | 6-CN | H | 3(D) | H | |
| 85 | 5-CF₃ | H | 3(D) | Ac | |
| 86 | 5-CF₃ | H | 3(D) | H | |
| 87 | H | H | 3(D) | -COOC₂H₅ | |
| 88 | 2-Cl | H | 3(D) | -COOC₂H₅ | |
| 89 | 6-CF₃ | H | 3(D) | -COOC₂H₅ | |
| 90 | 2-CN | H | 3(D) | -COOC₂H₅ | |
| 91 | H | H | 3(D) | -COCH₂-pip | |
| 92 | H | H | 3(D) | -COC₂H₅ | |
| 93 | H | H | 3(D) | -COC₃H₇ | |

| | | | | | |
|---|---|---|---|---|---|
| Ac = acétyle Bn = benzyl (Phénylméthyl) Pip = 1-piperazinyle Ms = méthane sulfonate Chlor = chlorhydrate Bromh = bromhydrate Quatern = sel de pyridinium quaternaire HSulf = hémisulfate Piv = pivalate | | | | | |

L'activité antithrombotique des composés selon l'invention a été étudiée *in vivo* chez le rat grâce à un test reproduisant une thrombose veineuse.

La thrombose veineuse a été induite selon le protocole décrit dans Thromb. Haemost. 1992, 67(1), 176-179. L'activité par voie intraveineuse ou par voie orale a été étudiée selon le protocole opératoire suivant :

L'expérimentation est réalisée sur des rats mâles Wistar, non à jeun; pesant 250 à 280 g et répartis en groupes de 10 animaux chacun. Les produits à tester sont administrés soit par voie orale (tubage) en solution ou en suspension dans du sérum physiologique, soit par injection intraveineuse, en solution dans du sérum physiologique ou dans un mélange PEG 400/eau. La concentration des composés est calculée de façon à faire absorber une quantité de solution de 2 ml/kg par voie orale et 1 ml/kg par injection intraveineuse. Une thrombose est induite à un temps T (0,5, 2, 4 ou 8 heures) après l'administration du produit et le thrombus formé est prélevé et pesé. Pour induire cette thrombose, on réalise une stase veineuse sous hypercoagulation, selon la technique décrite par WESSLER (J. Applied Physiol. 1959, 943-946) en utilisant en tant qu'agent hypercoagulant une solution de facteur X activé (Xa), fournie par la société Biogenic (Montpellier), et dosée à 7,5 nKat/kg. La stase veineuse est effectuée 15 secondes exactement après l'injection de l'agent hypercoagulant. L'activité des composés testés a été contrôlée à différentes doses, après qu'ils aient été administrés soit par voie orale (*p.o*.) soit par voie intraveineuse (*i.v*.). L'induction de la thrombose a été faite 2 heures ou 4 heures après l'administration du composé par voie orale et 2 heures après administration du composé par voie intraveineuse. A titre d'exemple, les résultats des tests précédents sont reportés dans les tableaux suivants pour quelques composés selon l'invention (l'activité est exprimée par le pourcentage d'inhibition de la formation du thrombus, observé en présence du composé selon l'invention, par rapport au poids du thrombus formé en l'absence du composé).

**Tableau I : Activité par voie orale**

| Exemple | Dose (mg/kg) | Temps (h) | Activité % |
|---|---|---|---|
| 1A | 6 | 4 | 51 |
| 2 | 3 | 4 | 91 |
| 2 | 1,5 | 4 | 67 |
| 2C | 6 | 4 | 55 |
| 2A | 4,2 | 2 | 96 |
| 12 | 3 | 4 | 52 |
| 18 | 3 | 4 | 46 |
| 20 | 3 | 4 | 50 |
| 31A | 5,6 | 4 | 63 |
| 31B | 8 | 4 | 94 |
| 36 | 6 | 2 | 91 |
| 37 | 6 | 2 | 96 |
| 41 | 6 | 2 | 97 |
| 49 | 6 | 2 | 90 |
| 93 | 6 | 2 | 81 |

**Tableau II : Activité par voie intraveineuse**

| Exemple | Dose (mg/kg) | Temps (h) | Activité % |
|---|---|---|---|
| 1A | 4 | 2 | 83 |
| 2 | 2,5 | 2 | 72 |
| 2A | 2,5 | 2 | 68 |
| 2A | 5 | 2 | 94 |
| 2C | 2,5 | 2 | 72 |
| 2C | 4 | 2 | 95 |
| 31B | 5 | 2 | 88 |

Ces résultats montrent que les composés selon l'invention présentent une activité antithrombotique veineuse, à la fois par voie orale et par voie intraveineuse.

La présente invention a donc pour objet un composé de formule (I) selon l'invention ainsi que ses sels avec un acide, solvates et hydrates pharmaceutiquement acceptables pour leur utilisation en tant que médicament. Le composé de formule (I) ou un de ses sels, solvates ou hydrates pharmaceutiquement acceptables pourra être utilisé pour la préparation d'un médicament antithrombotique destiné, en particulier, au traitement ou à la prévention des troubles de la circulation veineuse et notamment, pour corriger certains paramètres hématologiques sensibles au niveau veineux.

La présente invention a donc également pour objet des compositions pharmaceutiques contenant un composé de formule (I) ou un de ses sels, solvates ou hydrates pharmaceutiquement acceptables. Ces compositions pharmaceutiques contiennent en général des excipients convenables. Lesdits excipients sont choisis selon la forme pharmaceutique et le mode d'administration souhaités, en particulier orale ou injectable.

Ces compositions pharmaceutiques sont préparées selon les méthodes classiques bien connues de l'homme du métier. Par exemple, les composés selon l'invention peuvent être formulés avec des excipients physiologiquement acceptables pour obtenir une forme injectable à utiliser directement, une forme injectable à préparer extemporanément ou une forme solide pour administration par voie orale telle que, par exemple, une gélule ou un comprimé.

A titre d'exemple, une forme injectable peut être préparée de préférence par lyophilisation d'une solution filtrée et stérilisée contenant le composé selon l'invention et un excipient soluble en quantité nécessaire et suffisante pour obtenir une solution isotonique après addition extemporanée d'eau pour injection. Une forme administrable par voie orale sera de préférence présentée sous forme d'une gélule contenant le composé de l'invention broyé finement ou mieux, micronisé, et mélangé avec des excipients connus de l'homme du métier, tel que par exemple du lactose, de l'amidon prégélatinisé, du stéarate de magnésium.

Afin d'obtenir l'effet thérapeutique ou prophylactique désiré, chaque dose unitaire peut contenir 10 à 500 mg d'au moins un composé selon l'invention.

## Revendications

1. Composés du thioxylose, **caractérisés en ce qu'**ils sont choisis parmi :
a) les composés de formule : 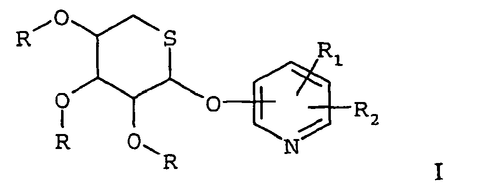 dans laquelle :
- le groupe pentapyranosyle représente un groupe 5-thio-β-D-xylopyranosyle ou un groupe 5-thio-β-L-xylopyranosyle,
- R représente un atome d'hydrogène, un groupe acyle en C₂-C₆, un groupe acétyle substitué par un hétérocycle azoté ou un groupe -COOR',
- R₁ et R₂ représentent chacun, indépendamment l'un de l'autre, un atome d'hydrogène, un atome d'halogène ou un groupe cyano, nitro, trifluorométhyle, un groupe alkyle en C₁-C₄ éventuellement substitué par un noyau aromatique, un groupe COOR', un groupe -CH₂-NR'R", un groupe alcoxy en C₁-C₄, un groupe - NH-CO-R', ou un groupe -NH-SO₂-R',
- R'et R" représentent chacun indépendamment un groupe alkyle en C₁-C₄,
b) leurs sels d'addition, oxydes ou sels d'ammonium quaternaires.

2. Composé selon la revendication 1, **caractérisé en ce que** le groupe pentapyranosyle représente un groupe 5-thio-β-D-xylopyranosyle ou un groupe 5-thio-β-L-xylopyranosyle,
R représente un atome d'hydrogène, un groupe acyle en C₂-C₆, ou un groupe -COOR',
R' représente un groupe alkyle en C₁-C₃,
R₁ et R₂ représentent chacun, indépendamment l'un de l'autre, un atome d'hydrogène, un atome d'halogène ou un groupe cyano, nitro, trifluorométhyle, ou un groupe alkyle en C₁-C₄ éventuellement substitué par un noyau aromatique.

3. Composé selon l'une des revendications 1 ou 2, **caractérisé en ce que** le groupe pentapyranosyle est le groupe 5-thio-β-D-xylopyranosyle.

4. Composé selon l'une quelconque des revendications 1 à 3, **caractérisé en ce que** le groupe pentapyranosyle est en position 3 de l'hétérocyle pyridine.

5. Composé selon l'une quelconque des revendications 1 à 4, **caractérisé en ce que** R₁ et R₂ représentent un atome d'hydrogène.

6. Composé selon l'une des revendications 1 à 5, **caractérisé en ce que** R représente un atome d'hydrogène.

7. Composé selon l'une des revendications 1 à 5, **caractérisé en ce que** R représente un groupe -COCH₃, un groupe -COOCH₃ ou un groupe COOC₂H₅.

8. Procédé de fabrication d'un composé selon l'une quelconque des revendications 1 à 7, **caractérisé en ce qu'**il comprend les étapes consistant à :
a) faire réagir un pyridinol de formule : 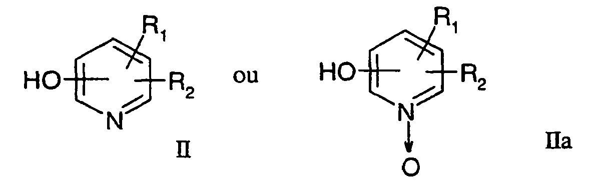 dans laquelle
- R₁ et R₂ représentent chacun, indépendamment l'un de l'autre, un atome d'hydrogène, un atome d'halogène ou un groupe cyano, nitro, trifluorométhyle, un groupe alkyle en C₁-C₄ éventuellement substitué par un noyau aromatique, un groupe COOR', un groupe -CH₂-NR'R", un groupe alcoxy en C₁-C₄, un groupe - NH-CO-R', ou un groupe -NH-SO₂-R',
- R'et R" représentent chacun indépendamment un groupe alkyle en C₁-C₄,
avec un dérivé du 5-thioxylopyranose de formule: 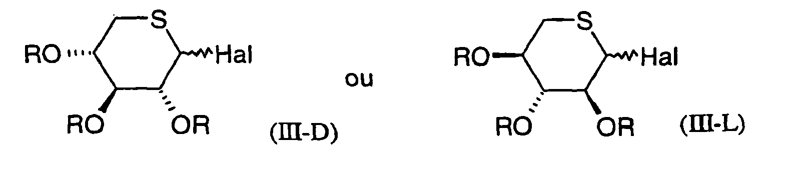 dans laquelle Hal représente un halogène, préférentiellement le brome et R représente un groupe acyle en C₂-C₆, dans un solvant aprotique, en présence d'un sel d'argent ou d'un sel de zinc en milieu anhydre, à une température comprise entre 25 et 80 °C et pendant 1 à 10 heures, pour obtenir le composé de formule I ou le N-oxyde correspondant: 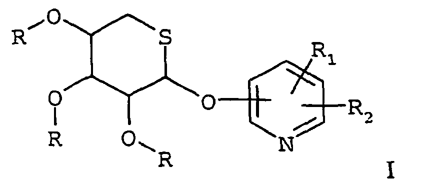 dans laquelle le groupe pentapyranose représente le D- ou le L-5-thioxylopyranose, et R, R₁, R₂ conservent la même signification que dans les composés de départ ;
b) si nécessaire, faire réagir le composé de formule I obtenu ci-dessus avec une solution d'ammoniac dans du méthanol pour obtenir le composé de formule: 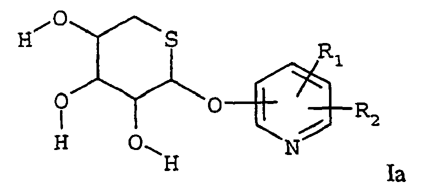 dans laquelle R₁ et R₂ conservent la même signification que ci-dessus ;
c) si nécessaire, faire réagir l'un des composés I ou Ia obtenus ci-dessus avec un acide pour obtenir le sel d'addition correspondant ;
d) si nécessaire, faire réagir l'un des composés de formule I ou Ia obtenus ci-dessus avec un halogénure organique pour obtenir le sel d'ammonium correspondant.

9. Procédé de fabrication d'un composé selon l'une quelconque des revendications 1 à 7, **caractérisé en ce qu'**il comprend les étapes consistant à:
a) faire réagir le tétra-O-acétyl-5-thioxylopyranose de formule : 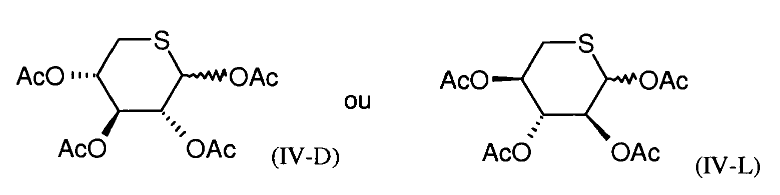 dans laquelle Ac représente le groupe acétyle avec un composé de formule : 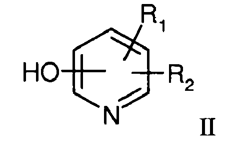 dans laquelle
- R₁ et R₂ représentent chacun, indépendamment l'un de l'autre, un atome d'hydrogène, un atome d'halogène ou un groupe cyano, nitro, trifluorométhyle, un groupe alkyle en C₁-C₄ éventuellement substitué par un noyau aromatique, un groupe COOR', un groupe -CH₂-NR'R", un groupe alcoxy en C₁-C₄, un groupe -NH-CO-R', ou un groupe -NH-SO₂-R',
- R'et R" représentent chacun indépendamment un groupe alkyle en C₁-C₄,
dans un solvant aprotique, en présence d'un catalyseur de type acide de Lewis, à une température comprise entre 20 et 60 °C et pendant 1 à 2 heures, pour obtenir le composé de formule : 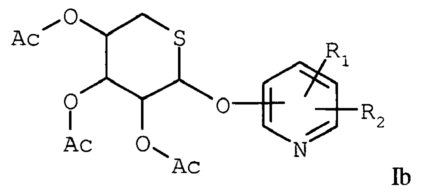 dans laquelle R₁ et R₂ conservent la même signification que dans les composés de départ ;
b) si nécessaire, faire réagir le composé de formule Ib obtenu ci-dessus avec le méthylate de sodium dans du méthanol pour obtenir le composé de formule : 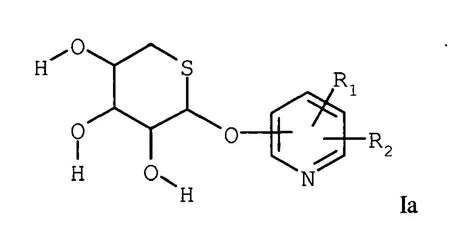 dans laquelle R₁ et R₂ conservent la même signification que ci-dessus;
c) si nécessaire, faire réagir l'un des composés Ib ou Ia obtenus ci-dessus avec un acide pour obtenir le sel d'addition correspondant.

10. Composé selon l'une quelconque des revendications 1 à 7 pour son utilisation en tant que médicament.

11. Utilisation d'un composé selon l'une quelconque des revendications 1 à 7 pour la préparation d'un médicament destiné à la prévention ou au traitement des thromboses, notamment les thromboses veineuses.

## Claims

1. Thioxylose compounds, **characterized in that** they are selected from:
a) the compounds of the formula: 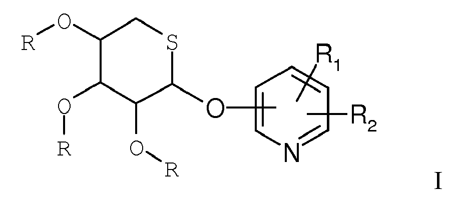 in which:
- the pentapyranosyl group is a 5-thio-β-D-xylopyranosyl group or a 5-thio-β-L-xylopyranosyl group,
- R is a hydrogen atom, a C₂-C₆ acyl group, an acetyl group substituted by a nitrogen heterocycle, or a group -COOR',
- R₁ and R₂ independently of one another are each a hydrogen atom, a halogen atom, a cyano, nitro or trifluoromethyl group, a C₁-C₄ alkyl group optionally substituted by an aromatic ring, a group -COOR', a group -CH₂-NR'R", a C₁-C₄ alkoxy group, a group -NH-CO-R' or a group -NH-SO₂-R', and
- R' and R" independently are each a C₁-C₄ alkyl group; and
b) their addition salts, oxides or quaternary ammonium salts.

2. Compound according to claim 1, **characterized in that** the pentapyranosyl group is a 5-thio-β-D-xylopyranosyl group or a 5-thio-β-L-xylopyranosyl group,
R is a hydrogen atom, a C₂-C₆ acyl group or a group -COOR',
R' is a C₁-C₃ alkyl group, and
R₁ and R₂ independently of one another are each a hydrogen atom, a halogen atom, a cyano, nitro or trifluoromethyl group or a C₁-C₄ alkyl group optionally substituted by an aromatic ring.

3. Compound according to claim 1 or 2, **characterized in that** the pentapyranosyl group is the 5-thio-β-D-xylopyranosyl group.

4. Compound according to any one of claims 1 to 3, **characterized in that** the pentapyranosyl group is in the 3-position of the pyridine heterocycle.

5. Compound according to any one of claims 1 to 4, **characterized in that** R₁ and R₂ are a hydrogen atom.

6. Compound according to one of claims 1 to 5, **characterized in that** R is a hydrogen atom.

7. Compound according to one of claims 1 to 5, **characterized in that** R is a group -COCH₃, a group -COOCH₃ or a group -COOC₂H₅.

8. Process for the manufacture of a compound according to any one of claims 1 to 7, **characterized in that** it comprises steps consisting in:
a) reacting a pyridinol of the formula: 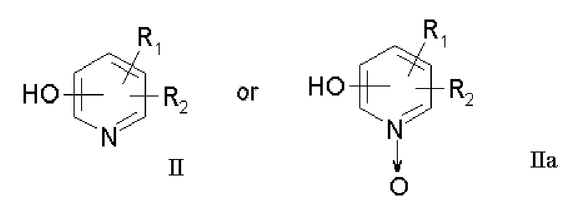 in which:
- R₁ and R₂ independently of one another are each a hydrogen atom, a halogen atom, a cyano, nitro or trifluoromethyl group, a C₁-C₄ alkyl group optionally substituted by an aromatic ring, a group -COOR', a group -CH₂-NR'R", a C₁-C₄ alkoxy group, a group -NH-CO-R' or a group -NH-SO₂-R', and
- R' and R" independently are each a C₁-C₄ alkyl group,
with a 5-thioxylopyranose derivative of the formula: 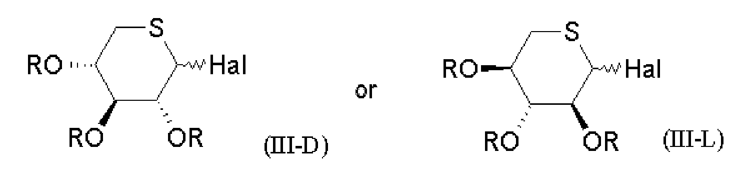 in which Hal is a halogen, preferably bromine, and R is a C₂-C₆ acyl group, in an aprotic solvent, in the presence of a silver salt or a zinc salt, in an anhydrous medium, at a temperature of between 25 and 80°C, for 1 to 10 hours, to give the compound of formula I or the corresponding N-oxide: 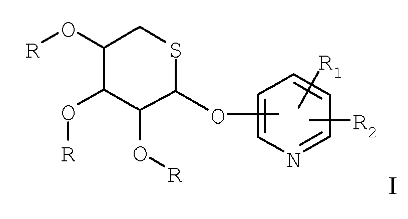 in which the pentapyranose group is D- or L-5-thioxylopyranose and R, R₁ and R₂ are as defined in the starting compounds;
b) if necessary, reacting the compound of formula I obtained above with a solution of ammonia in methanol to give the compound of the formula: 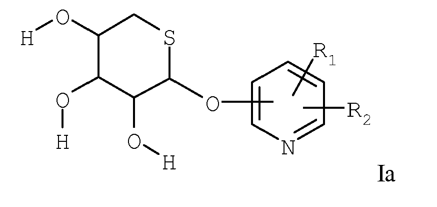 in which R₁ and R₂ are as defined above; and
c) if necessary, reacting one of the compounds obtained above, I or Ia, with an acid to give the corresponding addition salt; or
d) if necessary, reacting one of the compounds obtained above, of formula I or Ia, with an organic halide to give the corresponding ammonium salt.

9. Process for the manufacture of a compound according to any one of claims 1 to 7, **characterized in that** it comprises steps consisting in:
a) reacting the tetra-O-acetyl-5-thioxylopyranose of the formula: 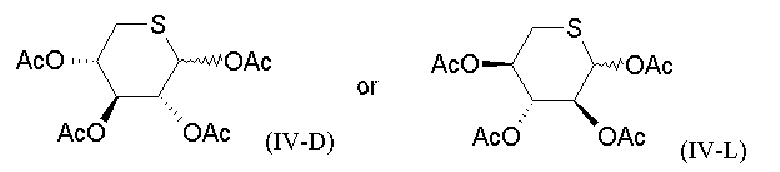 in which Ac is the acetyl group, with a compound of the formula: 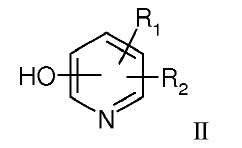 in which:
- R₁ and R₂ independently of one another are each a hydrogen atom, a halogen atom, a cyano, nitro or trifluoromethyl group, a C₁-C₄ alkyl group optionally substituted by an aromatic ring, a group -COOR', a group -CH₂-NR'R", a C₁-C₄ alkoxy group, a group -NH-CO-R' or a group -NH-SO₂-R', and
- R' and R" independently are each a C₁-C₄ alkyl group,
in an aprotic solvent, in the presence of a catalyst of the Lewis acid type, at a temperature of between 20 and 60°C, for 1 to 2 hours, to give the compound of the formula: 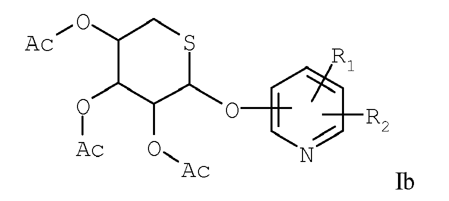 in which R₁ and R₂ are as defined in the starting compounds;
b) if necessary, reacting the compound of formula Ib obtained above with sodium methylate in methanol to give the compound of the formula: 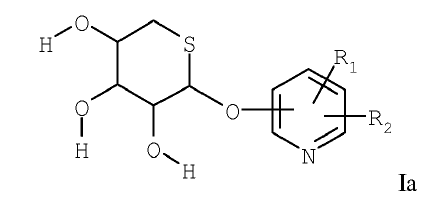 in which R₁ and R₂ are as defined above; and
c) if necessary, reacting one of the compounds obtained above, Ib or Ia, with an acid to give the corresponding addition salt.

10. Compound according to any one of claims 1 to 7 for its use as a drug.

11. Use of a compound according to any one of claims 1 to 7 for the preparation of a drug intended for the prevention or treatment of thromboses, especially venous thromboses.

## Patentansprüche

1. Thioxyloseverbindungen, **dadurch gekennzeichnet, daß** sie ausgewählt sind aus:
a) den Verbindungen der Formel: 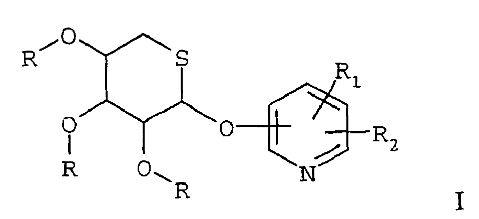 worin:
- die Pentapyranosyl-Gruppe eine 5-Thio-β-D-xylopyranosyl-Gruppe oder eine 5-Thio-β-L-xylopyranosyl-Gruppe darstellt,
- R ein Wasserstoffatom, eine C₂-C₆-Acylgruppe, eine Acetylgruppe, substituiert durch einen Stickstoff-Heterocyclus, oder eine -COOR'-Gruppe darstellt,
- R₁ und R₂ unabhängig voneinander jeweils ein Wasserstoffatom, ein Halogenatom oder eine Cyano-, Nitro-, Trifluormethylgruppe, eine C₁-C₄-Alkylgruppe, eventuell substituiert durch einen aromatischen Kern, eine COOR'-Gruppe, eine -CH₂-NR'R"-Gruppe, eine C₁-C₄-Alkoxygruppe, eine -NH-CO-R'-Gruppe oder eine -NH-SO₂-R'-Gruppe darstellen,
- R' und R" jeweils unabhängig eine C₁-C₄-Alkylgruppe darstellen,
b) ihren Additionssalzen, Oxiden oder quaternären Ammoniumsalzen.

2. Verbindung nach Anspruch 1, **dadurch gekennzeichnet, daß** die Pentapyranosyl-Gruppe eine 5-Thio-β-D-xylopyranosyl-Gruppe oder eine 5-Thio-β-L-xylopyranosyl-Gruppe darstellt.
- R ein Wasserstoffatom, eine C₂-C₆-Acylgruppe oder eine -COOR'-Gruppe darstellt,
- R' eine C₁-C₃-Alkylgruppe darstellt,
- R₁ und R₂ unabhängig voneinander jeweils ein Wasserstoffatom, ein Halogenatom oder eine Cyano-, Nitro-, Trifluormethylgruppe oder eine C₁-C₄-Alkylgruppe, eventuell substituiert durch einen aromatischen Kern darstellen.

3. Verbindung nach einem der Ansprüche 1 oder 2, **dadurch gekennzeichnet, daß** die Pentapyranosyl-Gruppe die 5-Thio-β-D-xylopyranosyl-Gruppe ist.

4. Verbindung nach einem der Ansprüche 1 bis 3, **dadurch gekennzeichnet, daß** die Pentapyranosyl-Gruppe in 3-Stellung des Pyridin-Heterocyclus ist.

5. Verbindung nach einem der Ansprüche 1 bis 4, **dadurch gekennzeichnet, daß** R₁ und R₂ ein Wasserstoffatom darstellen.

6. Verbindung nach einem der Ansprüche 1 bis 5, **dadurch gekennzeichnet, daß** R ein Wasserstoffatom darstellt.

7. Verbindung nach einem der Ansprüche 1 bis 5, **dadurch gekennzeichnet, daß** R eine -COCH₃-Gruppe, eine -COOCH₃-Gruppe oder eine COOC₂H₅-Gruppe darstellt.

8. Verfahren zur Herstellung einer Verbindung nach einem der Ansprüche 1 bis 7, **dadurch gekennzeichnet, daß** es die Schritte umfaßt, die darin bestehen:
a) ein Pyridinol der Formel: 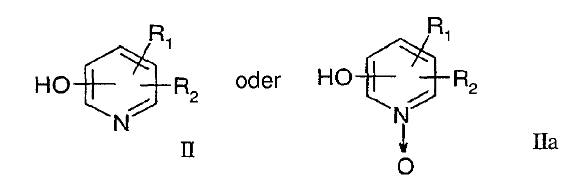 worin:
- R₁ und R₂ unabhängig voneinander jeweils ein Wasserstoffatom, ein Halogenatom oder eine Cyano-, Nitro-, Trifluormethylgruppe, eine C₁-C₄-Alkylgruppe, eventuell substituiert durch einen aromatischen Kern, eine COOR'-Gruppe, eine -CH₂-NR'R"-Gruppe, eine C₁-C₄-Alkoxygruppe, eine -NH-CO-R'-Gruppe oder eine -NH-SO₂-R'-Gruppe darstellen,
- R' und R" jeweils unabhängig eine C₁-C₄-Alkylgruppe darstellen, mit einem 5-Thioxylopyranose-Derivat der Formel: 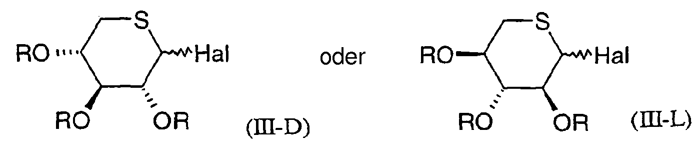
worin Hal ein Halogen, vorzugsweise Brom darstellt und R eine C₂-C₆-Acylgruppe darstellt, in einem aprotischen Lösungsmittel, in Anwesenheit eines Silbersalzes oder eines Zinksales, in wasserfreiem Medium, bei einer Temperatur zwischen 25 und 80 °C, für 1 bis 10 Stunde(n) reagieren zu lassen, um die Verbindung der Formel I oder das entsprechende N-Oxid zu erhalten: 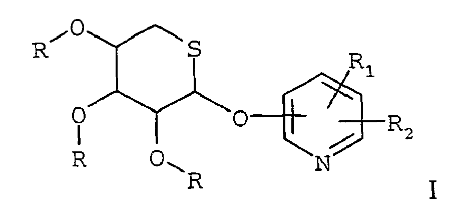 worin die Pentapyranose-Gruppe D- oder L-5-thioxylopyranose darstellt und R, R₁, R₂ die gleiche Bedeutung wie bei den Ausgangsverbindungen behalten,
b) falls erforderlich die oben erhaltene Verbindung der Formel I mit einer Ammoniaklösung in Methanol reagieren zu lassen, um die Verbindung folgender Formel zu erhalten: 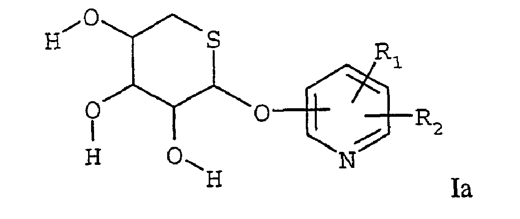 worin R₁ und R₂ die gleiche Bedeutung wie oben behalten,
c) falls erforderlich eine der oben erhaltenen Verbindungen I oder la mit einer Säure reagieren zu lassen, um das entsprechende Additionssalz zu erhalten,
d) fall erforderlich eine der oben erhaltenen Verbindungen der Formel I oder la mit einem organischen Halogenid reagieren zu lassen, um das entsprechende Ammoniumsalz zu erhalten.

9. Verfahren zur Herstellung einer Verbindung nach einem der Ansprüche 1 bis 7, **dadurch gekennzeichnet, daß** es die Schritte umfaßt, die darin bestehen:
a) Tetra-O-acetyl-5-thioxylopyranose der Formel: 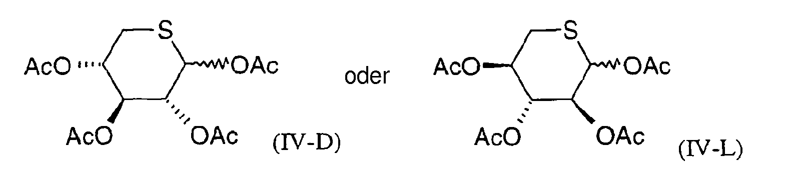 worin Ac die Acetylgruppe darstellt, mit einer Verbindung der Formel: 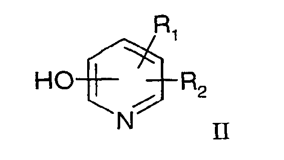 worin
- R₁ und R₂ unabhängig voneinander jeweils ein Wasserstoffatom, ein Halogenatom oder eine Cyano-, Nitro-, Trifluormethylgruppe, eine C₁-C₄-Alkylgruppe, eventuell substituiert durch einen aromatischen Kern, eine COOR'-Gruppe, eine -CH₂-NR'R"-Gruppe, eine C₁-C₄-Alkoxygruppe, eine -NH-CO-R'-Gruppe oder eine -NH-SO₂-R'-Gruppe darstellen,
- R' und R" jeweils unabhängig eine C₁-C₄-Alkylgruppe darstellen, in einem aprotischen Lösungsmittel, in Anwesenheit eines Katalysators vom Typ Lewis-Säure, bei einer Temperatur zwischen 20 und 60 °C, für 1 bis 2 Stunde(n) reagieren zu lassen, um die Verbindung folgender Formel zu erhalten: 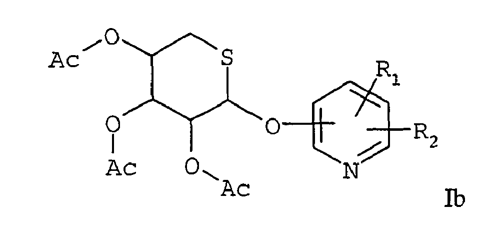
worin R₁ und R₂ die gleiche Bedeutung wie bei den Ausgangsverbindungen behalten,
b) falls erforderlich die oben erhaltene Verbindung der Formel Ib mit Natriummethylat in Methanol reagieren zu lassen, um die Verbindung folgender Formel zu erhalten: 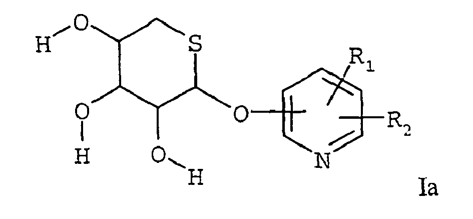 worin R₁ und R₂ die gleiche Bedeutung wie oben behalten,
c) falls erforderlich eine der oben erhaltenen Verbindungen Ib oder la mit einer Säure reagieren zu lassen, um das entsprechende Additionssalz zu erhalten.

10. Verbindung nach einem der Ansprüche 1 bis 7, für ihre Verwendung als Medikament.

11. Verwendung einer Verbindung nach einem der Ansprüche 1 bis 7, für die Herstellung eines Medikaments, das für die Prävention oder die Behandlung von Thrombosen, insbesondere Venenthrombosen bestimmt ist.
